# EUROPEAN PATENT APPLICATION

(11) **EP 4 375 280 A1**
(43) Date of publication of application: **29.05.2024**
(21) Application number: 22856214.6
(22) Date of filing: 10.08.2022
(51) Int. Cl.: C07D 403/14, C07D 207/00, A61K 31/409

(54) **METHOD FOR SYNTHESIZING BILIRUBIN**

(30) Priority: 11.08.2021 KR 20210106107
(71) Applicant: Bilix Co., Ltd., Seoul 06241 (KR)
(72) Inventor: KIM, Myung Lip, Busan 48272 (KR); MA, Sang Ho, Suwon-si, Gyeonggi-do 16438 (KR); PARK, Ki Soo, Suwon-si, Gyeonggi-do 16222 (KR); JUN, Hee Goo, Suwon-si, Gyeonggi-do 16508 (KR); KIM, Da Eun, Yongin-si, Gyeonggi-do 16944 (KR)
(74) Representative: BCKIP Part mbB
(86) International application number: PCT/KR2022/011915
(87) International publication number: WO 2023/018217

(57) **Abstract**

The present invention relates to a novel synthesis method of bilirubin. The method includes preparing a compound represented by Formula 3 by coupling a compound represented by Formula 1 with a compound represented by Formula 2, thereby firstly chemically synthesizing bilirubin and PEGylated bilirubin, which are usefully used in a medical product or the like.

## Description

### [Technical Field]

The present invention relates to a novel synthesis method of bilirubin.

### [Background Art]

Bilirubin is a component of bile and is mostly generated from hemoglobin in the body. Bilirubin is a yellowish final metabolite formed from heme, and despite many hydrophilic groups, it is extremely hydrophobic due to intramolecular hydrogen bonding.

Bilirubin was considered an undesirable substance as it caused jaundice when the blood level was high. However, in a recently published study, it was found that a slightly higher blood concentration of bilirubin significantly lowered the possibility of developing cardiovascular disease or cancer. Further, tissue-protecting effects of bilirubin were confirmed through animal experiments since it functions to remove different active oxygen and control immunocytes related to inflammation.

Although bilirubin is an industrially useful substance, it has been obtained by extracting from animals and has never been successfully synthesized. When bilirubin is extracted from animals, it is difficult to obtain bilirubin in large quantities while causing high production costs. Further, since bilirubin extracted from animals is a mixture of three regioisomers, it should undergo an additional separation and purification process in order to be utilized as a medicine. It is urgent to develop a method capable of chemically producing bilirubin.

### [Summary of Invention]

### [Problems to be Solved by Invention]

An object of the present invention is to provide a method for synthesizing bilirubin.

### [Means for Solving Problems]

1. A method for synthesizing bilirubin, including preparing a compound represented by Formula 3 below by coupling a compound represented by Formula 1 below with a compound represented by Formula 2 below:

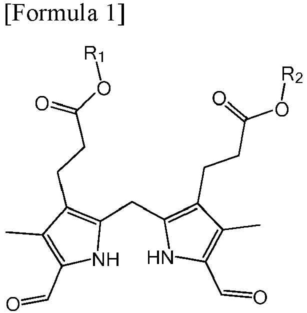

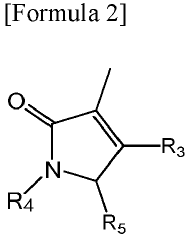

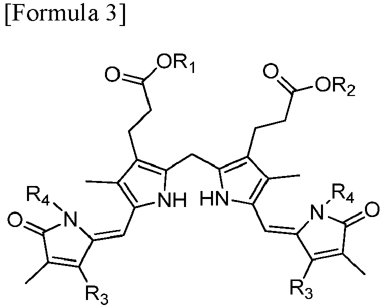

   (wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₃ is a vinyl or acetyl group, or an ethyl group substituted with a hydroxyl group, carbamate, selenide or sulfide; R₄ is hydrogen or a nitrogen protecting group; and R₅ is hydrogen, a tosyl or mesyl group).
2. The method according to the above 1, further including reacting the compound represented by Formula 3 with polyethylene glycol (PEG).
3. The method according to the above 1, wherein the compound represented by Formula 1 is reacted with polyethylene glycol (PEG) and then coupled with the compound represented by Formula 2.
4. The method according to the above 1, further including dimerizing a compound represented by Formula 7 below to prepare the compound represented by Formula 1:

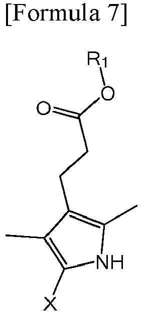

   (wherein, R₁ is the same as R₁ in Formula 1, X is an arylalkyl ester group having 8 to 20 carbon atoms, -CH₂OH, -COOH, a halogen atom or hydrogen).
5. The method according to the above 1, further including oxidizing a compound represented by Formula 9 below to prepare the compound represented by Formula 2:

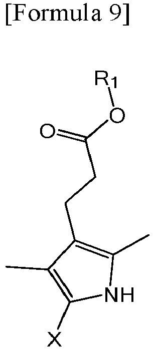

   (wherein, R₄ is the same as R₄ in Formula 2).
6. The method according to the above 1, further including reducing an acetyl group of a compound represented by Formula 10 below to prepare the compound represented by Formula 2: 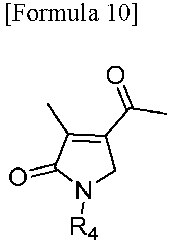 (wherein, R₄ is the same as R₄ in Formula 2).
7. The method according to the above 1, further including dehydrating a hydroxyl group of a compound represented by Formula 11 below to prepare the compound represented by Formula 2:

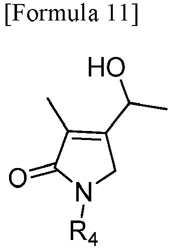

   (wherein, R₄ is the same as R₄ in Formula 2).
8. The method according to the above 1, further including processing a compound represented by Formula 12 through cyclization and halogen removal to prepare the compound represented by Formula 2:

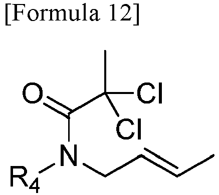

   (wherein, R₄ is the same as R₄ in Formula 2).
9. The method according to the above 1, further including oxidizing and carbamating a compound represented by Formula 13 below to prepare the compound represented by Formula 2: 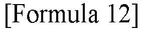 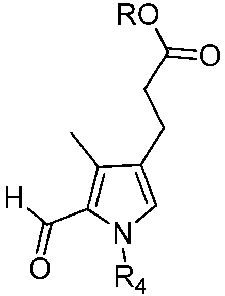 (wherein, R is an alkyl group having 1 to 12 carbon atoms, and R₄ is the same as R₄ in Formula 2).
10. The method according to the above 1, further including cyclizing a compound represented by Formula 14 below to prepare the compound represented by Formula 2:

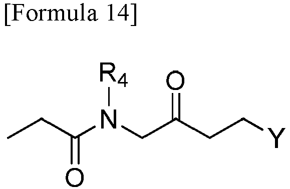

   (wherein, Y is selenide, and R₄ is the same as R₄ in Formula 2).
11. The method according to the above 1, further including oxidizing a compound represented by Formula 15 below to prepare the compound represented by Formula 2:

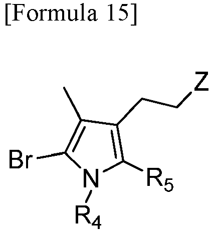

   (wherein, Z is sulfide, R₄ and R₅ are the same as R₄ and R₅ in Formula 2).
12. The method according to the above 1, further including preparing bilirubin from the compound represented by Formula 3.
13. The method according to the above 1, wherein the step of preparing a compound is carried out in the presence of a base selected from the group consisting of piperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidine-3-ol, piperazine, 2,6-dimethylpiperazine, 1-benzyl piperazine, 1-isopropyl piperazine, 2-ethyl piperazine, morpholine, 4-methyl morpholine, 2,6-dimethyl morpholine, ethyl morpholine, azepane, 2-methyl azepan, 4-methyl azepane, 2,2,7,7-tetramethyl azepane, 1,2,2-trimethyl azepane, 1,2-dimethylazepane, 2,7-dimethyl azepane, methyl azepane-4-carboxylate, azocane, 2-methyl azocane, 1,2-dimethyl azocane, 1,2,2-trimethyl azocane, methyl azocane-2-carboxylate, 1-methyl azocane and 2(2-methylphenyl)azocane.
14. The method according to the above 1, wherein the step of preparing a compound is carried out in the presence of a solvent selected from the group consisting of water, alcohols, ethers, ketones, aliphatic hydrocarbons, aromatic hydrocarbons, halogenated hydrocarbons, alkoxies, nitriles and amides.
15. The method according to the above 1, wherein the step of preparing a compound is carried out at -20 to 200 °C.
16. The method according to the above 1, wherein the step of preparing a compound is carried out for 0.5 to 120 hours.
17. The method according to the above 13, wherein the base is added in an amount of 2 to 20 moles based on 1 mole of the compound represented by Formula 1.

### [Advantageous effects]

The method for synthesizing bilirubin of the present invention may be economically performed under mild conditions.

The method for synthesizing bilirubin of the present invention has a high yield and is suitable for mass production.

### [Brief Description of Drawings]

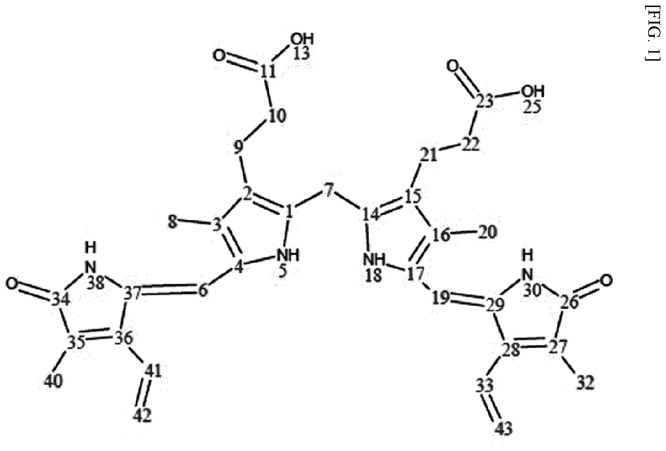

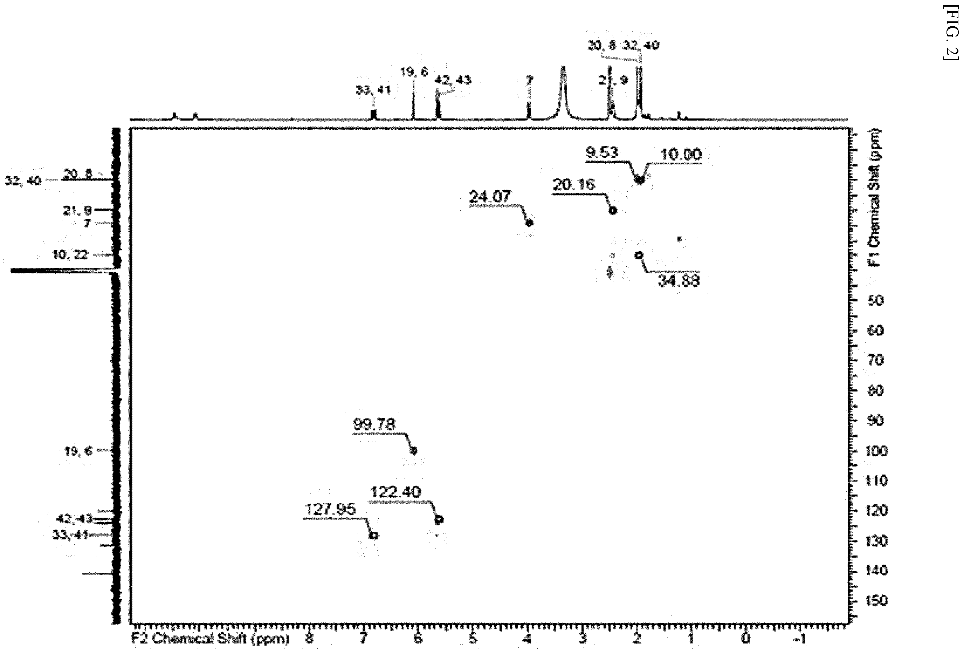

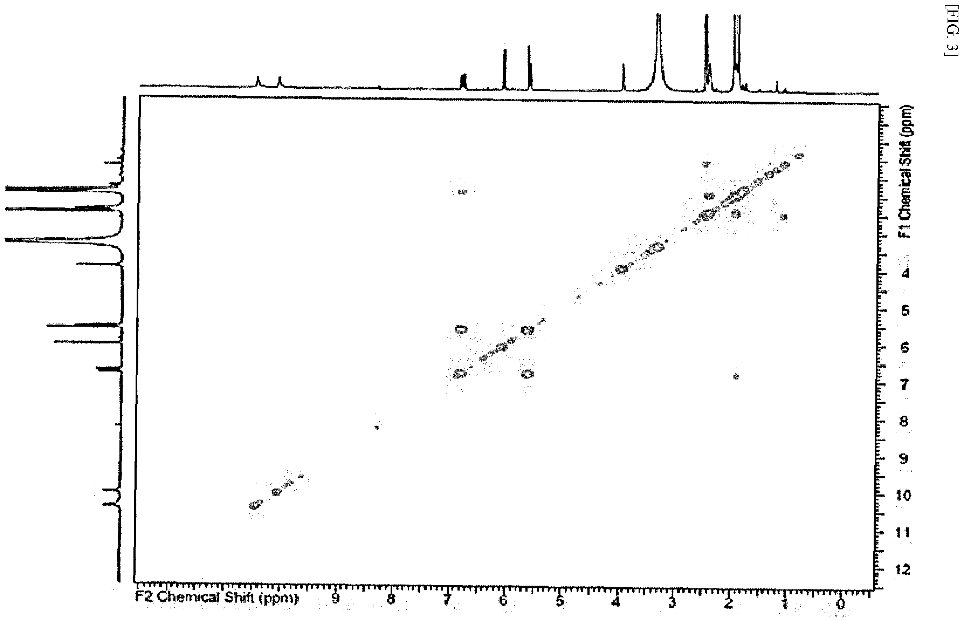

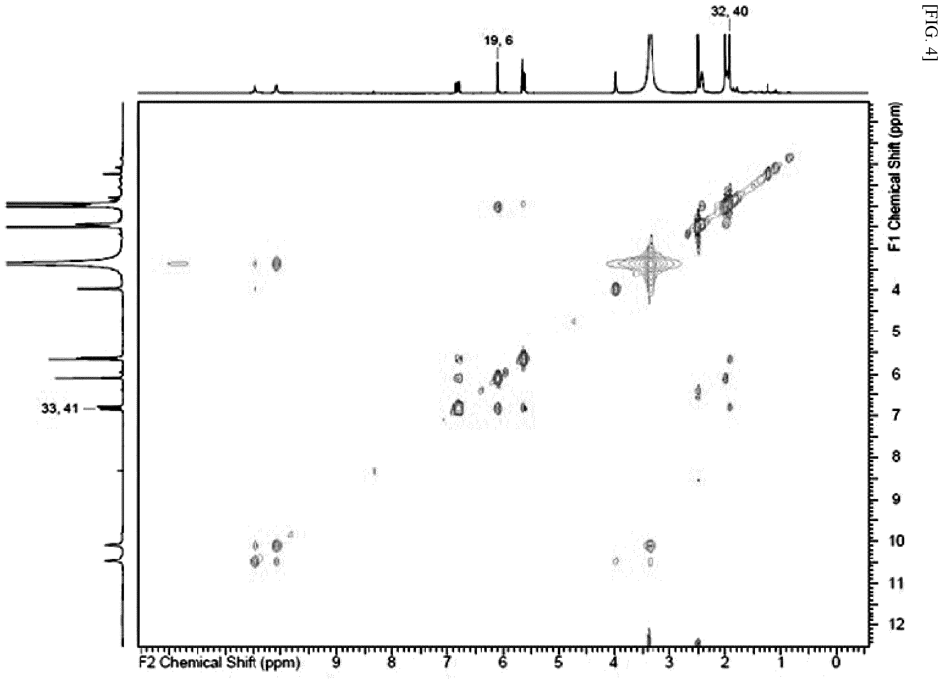

FIGS. 1 to 4 are 2D NMR data of Compound F-13a prepared in Example 13, wherein FIG. 2 is HSQC; FIG. 3 is COSY; and FIG. 4 is NOESY data.

### [Mode for Carrying out Invention]

The present invention relates to a novel method for synthesizing bilirubin.

As used herein, the term "alkyl" is a straight or branched, substituted or unsubstituted chain hydrocarbon, such as methyl, ethyl, n-propyl, isopropyl, cyclopropyl, n-butyl, sec-butyl, tert-butyl, cyclobutyl, cyclopropylmethyl, n-pentyl, isopentyl, neopentyl, tert-pentyl, cyclopentyl, cyclobutylmethyl, n-hexyl, isohexyl, cyclohexyl, cyclopentylmethyl and the like.

The term "cycloalkyl" is a monocyclic or bicyclic, substituted or unsubstituted cyclic hydrocarbon, such as cyclopropyl, cyclobutyl, cyclopentyl, cyclohexyl, cycloheptyl, cyclooctyl and the like.

The term "heterocycloalkyl" is a monocyclic or bicyclic, substituted or unsubstituted cyclic hydrocarbon containing one or more heteroatoms selected from B, N, O, S, P(=O), Si and P, such as tetrahydropyranyl, azetidyl, 1,4-dioxanyl, piperazinyl, piperidinyl, pyrrolidinyl, morpholinyl, thiomorpholinyl, dihydrofuranyl, dihydroimidazolyl, dihydroindolyl dihydroisooxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrothienyl, dihydrotriazolyl, dihydro-azetidyl, methylenedioxybenzoyl, tetrahydrofuranyl, tetrahydrothienyl and the like.

The term "aryl" is a monocyclic or bicyclic, substituted or unsubstituted aromatic group, such as phenyl, biphenyl, terphenyl, naphthyl, binapthyl, phenylnaphthyl, naphthylphenyl, phenylterphenyl, fluorenyl, phenylfluorenyl, diphenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, spirobifluorenyl, azyurenyl and the like.

"Aryl" includes, for example, phenyl, 1-naphthyl, 2-naphthyl, 1-anthryl, 2-anthryl, 9-anthryl, benzanthryl, 1-phenanthryl, 2-phenanthryl, 3-phenanthryl, 4-phenanthryl, 9-phenanthryl, naphthacetyl, pyrenyl, 1-chrysenyl, 2-chrysenyl, 3-chrysenyl, 4-chrysenyl, 5-chrysenyl, 6-chrysenyl, benzo[c]phenanthryl, benzo[g]chrysenyl, 1-triphenylenyl, 2-triphenylenyl, 3-triphenylenyl, 4-triphenylenyl, 1-fluorenyl, 2-fluorenyl, 3-fluorenyl, 4-fluorenyl, 9-fluorenyl, benzofluorenyl, dibenzofluorenyl, 2-biphenylyl, 3-biphenylyl, 4-biphenylyl, o-terphenyl, m-terphenyl-4-yl, m-terphenyl-3-yl, m-terphenyl-2-yl, p-terphenyl-4-yl, p-terphenyl-3-yl, p-terphenyl-2-yl, m-quaterphenyl, 3-fluoranthenyl, 4-fluoranthenyl, 8-fluoranthenyl, 9-fluoranthenyl, benzofluoranthenyl, o-tolyl, m-tolyl, p-tolyl, 2,3-xylyl, 3,4-xylyl, 2,5-xylyl, mesityl, o-cumenyl, m-cumenyl, p-cumenyl, p-tert-butylphenyl, p-(2-phenylpropyl)phenyl, 4'-methylbiphenylyl, 4"-tert-butyl-p-terphenyl-4-yl, 9,9-dimethyl-1-fluorenyl, 9,9-dimethyl-2-fluorenyl, 9,9-dimethyl-3-fluorenyl, 9,9-dimethyl-4-fluorenyl, 9,9-diphenyl-1-fluorenyl, 9,9-diphenyl-2-fluorenyl, 9,9-diphenyl-3-fluorenyl, 9,9-diphenyl-4-fluorenyl and the like.

The term "heteroaryl" refers to a monocyclic or bicyclic, substituted or unsubstituted aromatic group containing one or more heteroatoms selected from B, N, O, S, P(=O), Si and P, such as benzothienyl, benzoxazolyl, benzofuranyl, benzimidazolyl, benzthiazolyl, benzotriazolyl, sinnolinyl, furyl, imidazolyl, tetrazolyl, indazolyl, indolyl, isoxazolyl, isoquinolinyl, isothiazolyl, naphthyridinyl, oxadiazolyl, oxazolyl, isoxazolyl, purinyl, thiazolyl, isothiazolyl, thienopyridinyl, thienyl, thiadiazolyl, pyridinyl, pyridazinyl, pyrimidinyl, pyrazinyl, pyrazolyl, pyrrolyl, pyrido[2,3-d]pyrimidinyl, pyrrolo[2,3-b]pyridinyl, quinazolinyl, quinolinyl, thieno[2,3-c]pyridinyl, triazinyl and the like.

The term "arylalkyl" refers to an alkyl group in which at least one of the substituents is substituted with aryl, while "aryl" and "alkyl" are as defined above. For example, this includes benzyl, phenylethyl, phenylpropyl, phenylbutyl, phenylhexyl, naphthylethyl, naphthylpropyl, naphthylbutyl, naphthylhexyl, anthracenylmethyl, anthracenylethyl, anthracenylpropyl, anthracenylbutyl, phenanthrylmethyl, phenanthryl ethyl, phenanthrylpropyl, triphenylmethyl, triphenylethyl, triphenylpropyl, pyrenylmethyl, pyrenylethyl, pyrenylpropyl, phenylanthracenemethyl, phenylanthraceneethyl, phenylanthracenepropyl, perylenylmethyl, perylenylethyl, perylenylpropyl, chrysenylmethyl, chrysenylethyl, chrysenylpropyl, fluorenylmethyl, fluorenylethyl, fluorenylpropyl and the like.

The term "heteroarylalkyl" refers to an alkyl group in which at least one of the substituents is substituted with heteroaryl, while heteroaryl and alkyl are as defined above. For example, this includes pyridinylmethyl, pyridinylethyl, pyridinylpropyl, pyridinylbutyl, pyrimidinylmethyl, pyrimidinylethyl, pyrimidinylpropyl, pyrazolylmethyl, pyrazolylethyl, pyrazolylmethyl, pyrazolylethyl, pyrazolylpropyl, quinolinylmethyl, quinolinylethyl, quinolinylpropyl and the like.

The term "substituted" refers to inclusion of at least one substituent, for example, one or two or more of halogen atom, nitro, hydroxyl, cyano, amino, thiol, carboxyl, amide, nitrile, sulfide, disulfide, sulfenyl, formyl, formyloxy, formylamino, aryl or substituted aryl.

In the case where no substituent is described in a formula of the present invention even though it is a site requiring a substituent, it is considered that a hydrogen substituent is omitted.

The present invention relates to a method for synthesizing bilirubin, which includes preparing a compound represented by Formula 3 below by coupling a compound represented by Formula 1 below with a compound represented by Formula 2 below.

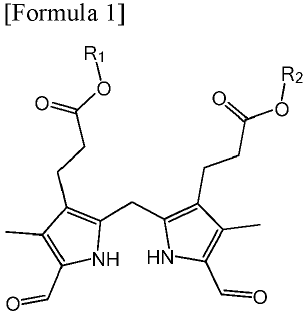

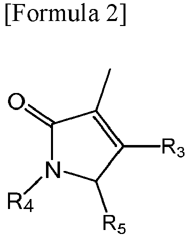

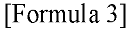

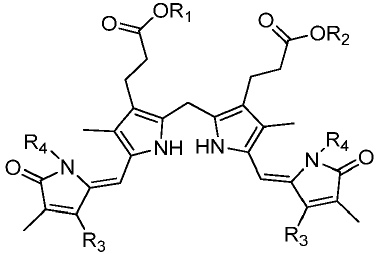

Wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms.

The number of carbon atoms in R₁ and R₂ may be appropriately selected within a range that does not affect a coupling reaction between the compound represented by Formula 1 and the compound represented by Formula 2.

For example, R₁ and R₂ may be each independently an alkyl group having 1 to 10 carbon atoms, an aryl group having 6 to 10 carbon atoms, a heteroaryl group having 2 to 10 carbon atoms, an arylalkyl group having 7 to 10 carbon atoms, or a heteroarylalkyl group having 3 to 10 carbon atoms.

Further, R₁ and R₂ may be each independently selected from an alkyl group having 1 to 5 carbon atoms, an aryl group having 6 to 10 carbon atoms, a heteroaryl group having 4 to 10 carbon atoms, an arylalkyl group having 7 to 10 carbon atoms, or a heteroarylalkyl group having 5 to 10 carbon atoms.

R₃ is a vinyl or acetyl group; or an ethyl group substituted with a hydroxyl group, carbamate, selenide or sulfide.

Herein, the carbamate is a functional group having a structure of Formula 4 below.

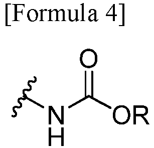

In Formula 4, R is an alkyl group having 1 to 12 carbon atoms or alkyl having 1 to 5 carbon atoms.

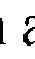

Selenide is a functional group having a structure of Formula 5 below, while sulfide is a functional group having a structure of Formula 6 below.

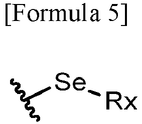

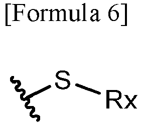

Wherein, in Formulas 5 and 6, Rx may be hydrogen, or a substituted or unsubstituted, straight-chain or branched alkyl, cycloalkyl, heterocycloalkyl, aryl, heteroaryl, arylalkyl, or heteroarylalkyl group.

For example, Rx is an alkyl group having 1 to 12 carbon atoms, a cycloalkyl group having 5 to 20 carbon atoms, a heterocycloalkyl group having 2 to 20 carbon atoms, an aryl group having 5 to 20 carbon atoms, a heteroaryl group having 2 to 20 carbon atoms, an arylalkyl group having 6 to 20 carbon atoms, or a heterocycloalkyl group having 3 to 20 carbon atoms.

For example, Rx is a phenyl or p-tolyl group.

R₃ may be an ethyl group substituted with a hydroxyl group, for example, may be a functional group in which a hydroxyl group is substituted at the position of carbon No. 1 of an ethyl group.

R₃ may be an ethyl group substituted with carbamate. For example, it is a functional group in which a carbamate is substituted at the position of carbon No. 2 of an ethyl group.

R₃ may be an ethyl group substituted with selenide, for example, may be a functional group in which selenide is substituted at the position of carbon No. 2 of an ethyl group.

R₃ may be an ethyl group substituted with sulfide, for example, may be a functional group in which sulfide is substituted at the position of carbon No. 2 of an ethyl group.

R₄ is hydrogen or a nitrogen protecting group.

Herein, the nitrogen-protecting group is not limited to a specific one as long as it is a substituent capable of protecting the nitrogen atom to which R₄ is bonded, and for example, may be selected from the group consisting of -COORₓ (Rₓ is as defined above), tert-butyloxycarbonyl (Boc), trityl (-CPh₃), tosyl group (SOOPhCH₃), 9-fluorenylmethyloxy carbonyl (Fmoc), carboxybenzyl group (Cbz), p-methoxybenzyl carbonyl (Moz), acetyl (Ac), benzoyl (Bz), p-methoxybenzyl (PMB), 3,4-dimethoxybenzyl (DMPM), p-methoxyphenyl (PMP), 2-naphthylmethyl ether (Nap), and trichloroethyl chloroformate (Troc).

R₅ is hydrogen, a tosyl ( 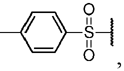 Ts or Tos) or methyl group ( 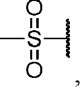 Ms).

When the nitrogen-protecting group of R₄ of Formula 2 remains after the compound represented by Formula 1 and the compound represented by Formula 2 are coupled, a step of removing the nitrogen-protecting group may be additionally required.

The compound represented by Formula 1 and the compound represented by Formula 2 may be bonded in a molar ratio of 1:2.

The compound represented by Formula 1 and the compound represented by Formula 2 may be introduced at a molar ratio of 1: 2 to 10, 1: 2 to 5, 1: 2 to 4, or 1: 2 to 3 depending on the reaction.

The coupling reaction is carried out in the presence of a solvent and base.

The solvent is an inorganic solvent or an organic solvent. The organic solvent may be, for example, alcohols, ethers, ketones, aliphatic hydrocarbons, aromatic hydrocarbons, halogenated hydrocarbons, alkoxies, nitriles or amides. Solvents belonging to these classes are, for example, listed in Table 1. The inorganic solvent is, for example, water.

**[TABLE 1]**

| Division | Organic solvent |
|---|---|
| Alcohols | Methanol, ethanol, propanol, isopropanol, ethylene glycol |
| Ethers | Diethylether, tetrahydrofuran(THF), 2-methyl tetrahydrofuran, dioxanes |
| Ketones | Methyl cellosolve, ethyl cellosolve, butyl cellosolve, methylethyl ketone, acetone |
| Aliphatic hydrocarbons | Hexane, heptane, octane |
| Aromatic hydrocarbons | Benzene, toluene, xylene |
| Halogenated hydrocarbons | Dichloromethane (DCM), chloroform, chlorobenzene |
| Alkoxies | Methoxyethane, dimethoxyethane (DME), methoxypropane, dimethoxypropane |
| Nitriles | Acetonitrile, benzonitrile, trinitrile |

The base is an organic base or an inorganic base.

The base is preferably a stronger base than the compound represented by Formula 2. As the organic base, it is preferable to use an amine organic base. For example, it may be chain type amine organic bases such as methylamine, ethylamine, dimethylamine, diethylamine, ethylmethylamine, propylamine, dipropylamine, methylpropylamine, ethylpropylamine, diisopropylamine, N-methylcyclohexylamine or trimethylamine, etc.; or cyclic amine organic bases such as aziridine, azetidine, oxaziridine, azetidine, diazetidine, imidazolidine, pyrazolidine, oxazolidine, isoxazolidine, thiazolidine, isothiazolidine, piperidine, 2-methylpiperidine, 2-ethylpiperidine, 2,6-dimethylpiperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidin-3-ol, piperazine, 2,6-dimethylpiperazine, 1-benzylpiperazine, 1-isopropylpiperazine, 2-ethylpiperazine, N-propylpiperazine, morpholine, thiomorpholine, 4-methyl morpholine, 2,6-dimethyl morpholine, ethyl morpholine, azepane, 2-methyl azepane, 4-methyl azepane, 2,2,7,7-tetramethyl azepane, 1,2,2-trimethyl azepane, 1,2-dimethyl azepane, 2,7-dimethyl azepane, azocane, 1,2-dimethyl azocane, 1,2,2-trimethyl azocane, methyl azocane-2-carboxylate, 1-methyl azocane, 2-(2-methylphenyl) azocane or proline, etc.

The organic base is preferably piperidine, pyrrolidine, morpholine, piperazine, azepane, azocane, N-methylpiperidine, N-ethylpiperidine or proline.

The inorganic base may be, for example, LiOH, KOH or NaOH.

The base may be included in an amount of 2 to 20 moles, 2 to 15 moles, 2 to 10 moles, 4 to 20 moles, 4 to 15 moles, 4 to 10 moles, or 5 to 20 moles, 5 to 15 moles, 5 to 10 moles, 6 to 20 moles, 6 to 15 moles or 6 to 10 moles based on 1 mole of the compound represented by Formula 1.

The coupling reaction temperature of the present invention is -20 to 200 °C. For example, it may be 30 to 180 °C, 30 to 150 °C, 30 to 120 °C, 30 to 100 °C, 40 to 150 °C, 40 to 140 °C, 40 to 120 °C, 40 to 100 °C, 50 to 150 °C, 50 to 120 °C, or 50 to 100 °C. The optimum reaction temperature may vary depending on the solvent and base used.

The coupling reaction time of the present invention may be 10 minutes to 120 hours, for example, 1 to 72 hours, 1 to 48 hours, 1 to 24 hours, 3 to 72 hours, 3 to 48 hours, 3 to 24 hours, 6 to 72 hours, 6 to 48 hours, or 6 to 24 hours. The optimal reaction time may vary depending on the solvent and base used.

The method for synthesizing bilirubin of the present invention may further include converting R₁ and/or R₂ of the compound represented by Formula 3 into hydrogen through a saponification reaction. For example, when R₁ and R₂ of the compound represented by Formula 3 are a methyl group, a base such as LiOH, KOH or NaOH may be added to the compound represented by Formula 3 in order to replace the methyl group with hydrogen.

The solvent used for the saponification reaction is not particularly limited. As the solvent for saponification, the same solvent as for the coupling reaction may be used. For example, it may be methanol, ethanol, 2-propanol, tetrahydrofuran (THF), 2-methyltetrahydrofuran (2-MeTHF), dioxane, acetonitrile, N,N-dimethylformamide (DMF), t-butanol, dimethoxyethane (DME), dichloromethane (DCM), isopropyl alcohol or the like.

The saponification may be carried out under conditions known in the art. For example, it may be performed at 10 to 150 °C for 1 to 72 hours, or at 10 to 60 °C for 1 to 48 hours.

The method for synthesizing bilirubin of the present invention may further include a PEGylation step of reacting the compound represented by Formula 3 with polyethylene glycol (PEG).

The method for synthesizing bilirubin of the present invention may include a step of PEGylating the compound represented by Formula 1 with polyethylene glycol (PEG) and then coupling the resulting product with the compound represented by Formula 2.

PEGylated bilirubin has improved water solubility.

Polyethylene glycol is, for example, mPEGₙ-NH₂ (methoxypolyethylene glycol-amine, n=5 to 60). Herein, n is the number of -CH₂-CH₂-O- repeating units of methoxypolyethylene glycol-amine, which may range from 5 to 60, 10 to 50, 10 to 40, 20 to 40, 10 to 30, or 20 to 30.

PEGylation includes mono-PEGylation in which either O-R₁ or O-R₂ is PEGylated, and bi-PEGylation in which both of them are PEGylated.

In the PEGylation reaction, polyethylene glycol may be added in an appropriate amount in consideration of the number of moles of the compound represented by Formula 1 or Formula 3. For example, polyethylene glycol may be added in an amount of 0.1 to 10 moles, 0.1 to 8 moles, 0.1 to 5 moles, 0.3 to 8 moles, 0.3 to 5 moles, or 0.3 to 4 moles, or 0.3 to 3 moles based on 1 mole of the compound represented by Formula 1 or Formula 3.

As reagents for PEGylation reaction, CDI (1,1-carbonyldiimidazole), CMPI (2-chloro-1-methylpyridinium iodide), BEP (2-bromo-1-ethyl-pyridinium tetrafluoroborate), EDCI (1-ethyl-3-(3-dimethylaminopropyl)carbodiimide), HATU (1-[bis(dimethylamino)methylene]-1H-1,2,3-triazolo[4,5-b]pyridinium 3-oxide hexafluorophosphate; hexafluorophosphate azabenzotriazole tetramethyl uronium, DCC (N,N'-dicyclohexylcarbodiimide) or HOBt (hydroxybenzotriazole), etc. may be used, but it is not limited thereto.

The reagent for PEGylation reaction may be added in an amount of 0.3 to 5 moles, 0.3 to 3 moles, 0.5 to 5 moles, 0.5 to 3 moles, 0.5 to 2.5 moles or 0.5 to 2 moles based on 1 mole of the compound represented by Formula 1 or Formula 3, but it is not limited thereto.

The solvent for PEGylation reaction is not particularly limited. As the solvent for PEGylation reaction, the same solvent as for coupling reaction may be used. For example, it may be DMSO (dimethyl sulfoxide), DMF (dimethylformamide), DMA (dimethylacetamide) or pyridine.

The PEGylation reaction may be carried out in the presence of a base. The base may be selected within the range previously exemplified as the bases in the coupling reaction, and is preferably DIPEA (N,N-Diisopropylethylamine) or pyridine.

The PEGylation reaction may be carried out at 10 to 100 °C, such as 10 to 80 °C, 20 to 60 °C, 20 to 50 °C, or 20 to 30 °C.

The PEGylation reaction may be carried out for 1 to 24 hours, 1 to 18 hours, and 1 to 12 hours, but it is not limited thereto.

In one embodiment, the PEGylation reaction may be performed by adding 0.3 to 5 moles of polyethylene glycol and 0.5 to 5 moles of a coupling reagent (CDI, EDCI, CMPI, etc.) based on 1 mole of the compound represented by Formula 1 or Formula 3, and this reaction may be carried out at 20 to 40 °C for 0.5 to 24 hours.

The compound represented by Formula 1, as well as the compound represented by Formula 2 as reactants in the bilirubin synthesis method of the present invention may be prepared as follows.

The compound represented by Formula 1 may be prepared by dimerizing a compound represented by Formula 7 below and replacing X of the product with -C(=O)H.

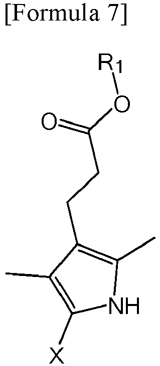

Wherein R₁ in Formula 7 is the same as R₁ in Formula 1, and X is an arylalkyl ester group having 8 to 20 carbon atoms, -CH₂OH, -COOH, a halogen atom, or hydrogen.

The arylalkyl ester group is a functional group in which R_{Y} in Formula 8 below is an arylalkyl group.

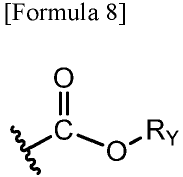

The arylalkyl group of Formula 8 is the same as the arylalkyl group of Formula 1.

The number of carbon atoms in the arylalkyl ester group may be appropriately selected within a range that does not affect the dimerization of the compound represented by Formula 7. For example, it may have 8 to 20 carbon atoms, 8 to 18 carbon atoms, 8 to 15 carbon atoms, or 8 to 12 carbon atoms.

A method for substituting X of the product with an aldehyde group is, for example, as follows (1) to (5).
(1) When X is an arylalkyl ester group, the arylalkyl ester group is reduced to -COOH by hydrogenation, then -COOH is reduced or removed. Thereafter, -C(=O)H may be added to replace X with an aldehyde group.
   In one embodiment, the hydrogenation may be carried out under a Pd/C catalyst. For example, the arylalkyl ester group is -C(=O)OBn (Bn=benzyl), then -C(=O)OBn is reduced to - COOH by a hydrogenation reaction under a Pd/C catalyst. Thereafter, -COOH is removed to replace the arylalkyl ester group with -C(=O)H.
(2) When X is -CH₂OH, X is oxidized by a known method and replaced with -C(=O)H.
(3) When X is -COOH, -COOH is reduced or removed, followed by adding -C(=O)H to replace X with an aldehyde group.
(4) When X is a halogen atom, the halogen atom is substituted with an aldehyde group by a carbonylation reaction. A known method can be used for the carbonylation reaction. For example, the carbonylation reaction may be carried out using carbon monoxide and palladium.
(5) When X is hydrogen, hydrogen is replaced with an aldehyde group by an aldehyde addition reaction. A known method may be used for the aldehyde addition reaction. For example, an aldehyde addition reaction may be performed using BuLi and DMF.

The dimerization of the compound represented by Formula 6 may be carried out under, for example, bromine (Br₂) conditions. A solvent for dimerization is not particularly limited. For example, as the organic solvent, the solvents in Table 1 exemplified as the coupling reaction solvents may be used.

The dimerization reaction may be performed at 10 to 100 °C, for example, 10 to 80 °C, 20 to 60 °C, 20 to 50 °C, or 20 to 30 °C.

The dimerization reaction may be performed for 1 to 24 hours, 1 to 18 hours, or 1 to 12 hours, but it is not limited thereto.

The compound represented by Formula 2 (R₃ = acetyl) may be prepared by oxidizing the compound represented by Formula 9.

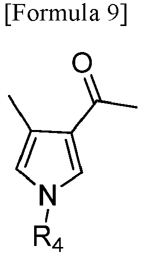

Wherein R₄ is the same as R₄ in Formula 2.

The oxidation reaction of the compound represented by Formula 9 may be performed in the presence of H₂O₂ and pyridine. The oxidation reaction of the compound represented by Formula 9 may be carried out within a range of the solvent, temperature, time, etc. for the coupling reaction, but it is not limited thereto.

The compound represented by Formula 2 (R₃ = an ethyl group substituted with a hydroxyl group) may be prepared by reducing the acetyl group of a compound represented by Formula 10 below. 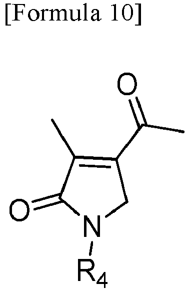 (wherein, R₄ is the same as R₄ in Formula 2).

The reaction of reducing the acetyl group may be performed by a known method, and the conditions for the same may be implemented within a range of solvent, temperature, time, and the like, in the coupling reaction. For example, the acetyl group may be reduced using MeOH as a solvent and NaBH₄ and CeCl₃·7H₂O as a reduction reaction reagent, or THF as a solvent and DIBAL as a reduction reaction reagent.

The compound represented by Formula 2 (R₃ = a vinyl group) may be prepared by dehydrating the hydroxyl group of a compound represented by Formula 11 below.

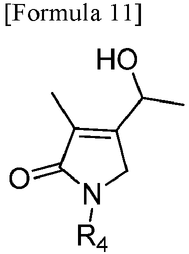

Wherein R₄ is the same as R₄ in Formula 2.

The reaction of dehydrating the hydroxyl group may be performed by a known method, and the conditions may be performed within a range of the solvent, temperature, time, and the like in the previous coupling reaction. For example, the hydroxyl group may be dehydrated using DCM (dichloromethane) as a solvent and POCl₃ and TEA as reaction reagents.

The compound represented by Formula 2 (R₃ = a vinyl group) may be prepared through cyclization and halogen removal reactions of a compound represented by Formula 12.

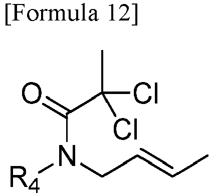

Wherein R₄ is the same as R₄ in Formula 2.

The cyclization reaction of the compound represented by Formula 12 may be performed under CuCl and acetonitrile (ACN), and the halogen removal reaction may be performed under DMF. The cyclization and halogen removal reactions of the compound represented by Formula 12 may produce a compound represented by Formula 2 below.

The compound represented by Formula 2 (R₃= an ethyl group substituted with carbamate) may be prepared by oxidizing and carbamating the compound represented by Formula 13.

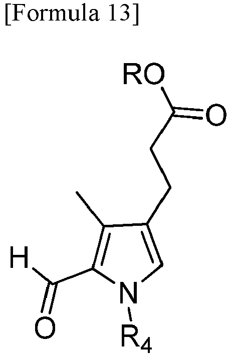

Wherein R₄ is the same as R₄ in Formula 2, and R is the same as R in Formula 4.

Oxidation and carbamation of the compound represented by Formula 13 may be performed in the following steps.

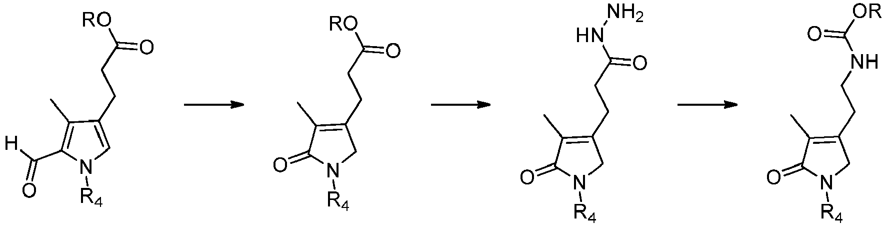

The oxidation reaction of the compound represented by Formula 13 may be carried out, for example, in the presence of H₂O₂ and pyridine. Thereafter, the carbamation may be carried out, for example, by treatment with NH₂NH₂ and then with NaNO₂/HCl and an alcohol. Each reaction may be performed within a range of solvent, temperature, time, etc. for the coupling reaction, but it is not limited thereto.

The compound represented by Formula 2 (R₃= an ethyl group substituted with selenide) may be prepared by cyclizing the compound represented by Formula 14.

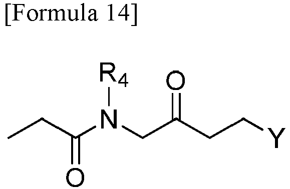

Wherein Y is selenide, and R₄ is the same as R₄ in Formula 2.

The cyclization reaction of the compound represented by Formula 14 may be performed under t-BuOK, and may be performed within a range of solvent, temperature, time, etc. for the coupling reaction. When the compound represented by Formula 14 is cyclized, a compound represented Formula 2 below is prepared.

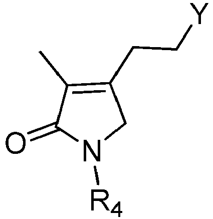

The compound represented by Formula 2 (R₃= an ethyl group substituted with sulfide) may be prepared by oxidizing the compound represented by Formula 15.

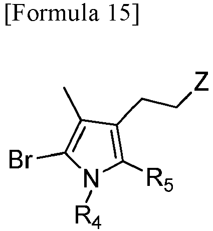

Wherein Z is sulfide, and R₄ and R₅ are the same as R₄ and R₅ in Formula 2.

The compound represented by Formula 2 obtained by oxidizing the compound represented by Formula 15 (R₃ = an ethyl group substituted with sulfide) is as follows.

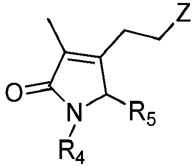

The oxidation reaction of the compound represented by Formula 15 may be performed by treatment with TFA/H₂O. The oxidation reaction of the compound represented by Formula 15 may be performed within a range of a solvent, temperature, time, etc. for the coupling reaction.

When R₅ is a tosyl or mesyl group, a coupling reaction with the compound represented by Formula 1 may be carried out after removing the tosyl group. Removal of the tosyl group or mesyl group may be performed by a known method, for example, by treatment with NaBH₄.

When R₃ of the compound represented by Formula 3 of the present invention is an acetyl group, or an ethyl group substituted with a hydroxyl group, carbamate, selenide or sulfide, a step of converting these substituents to a vinyl group should be additionally performed.
(1) When R₃ is an acetyl group, conversion to a vinyl group may be carried out by reduction and dehydration of the acetyl group. Reduction and dehydration of acetyl groups may be performed by known methods.
(2) When R₃ is an ethyl group substituted with a hydroxyl group, conversion to a vinyl group may be carried out by a dehydration reaction of the hydroxyl group. The dehydration reaction of the hydroxyl group may be performed by a known method.
(3) When R₃ is an ethyl group substituted with carbamate, conversion to a vinyl group may be carried out by Hofmann elimination reaction after deprotection reaction. For example, the compound represented by Formula 3 (Compound D-Gd) may be converted into a vinyl group (Compound F-13a) through removal of the protecting group and Hoffman elimination reaction in the presence of LiOH as follows. 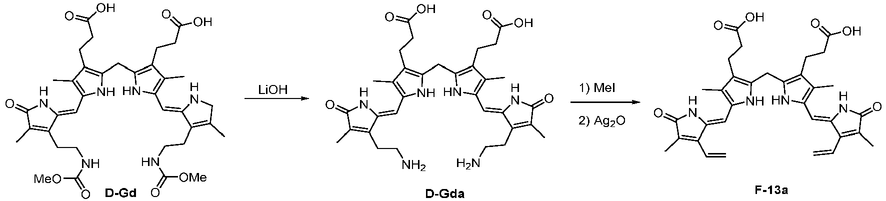
(4) When R₃ is an ethyl group substituted with selenide, conversion to a vinyl group may be carried out by an oxidation reaction of selenide. For example, the compound of Formula 3 may be converted into a vinyl group by oxidation in the presence of HOAc and H₂O₂ as follows. 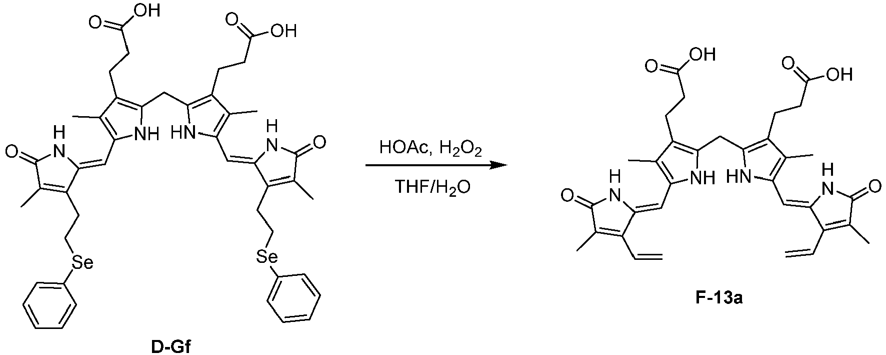
(5) When R₃ is an ethyl group substituted with sulfide, conversion to a vinyl group may be carried out by an oxidation reaction of sulfide. For example, the compound of Formula 3 may be oxidized with mCPBA and then deprotected with pyridine as follows.

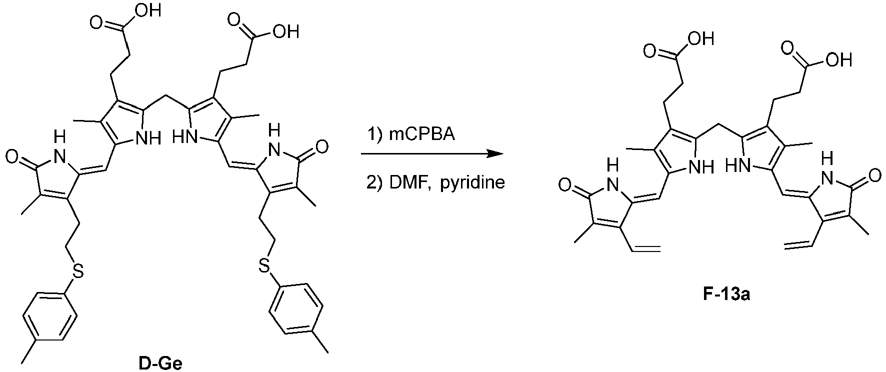

Hereinafter, the present invention will be described in more detail through examples.

### <EXAMPLE>

### 1. Preparation of the compound represented by Formula 1

Compound C, Compound D, Compound C1 and Compound C2 corresponding to the compound represented by Formula 1 herein were prepared as follows (Examples 1 to 4).

### Example 1: Preparation of Compound C

### (1-1) Preparation of Compound A

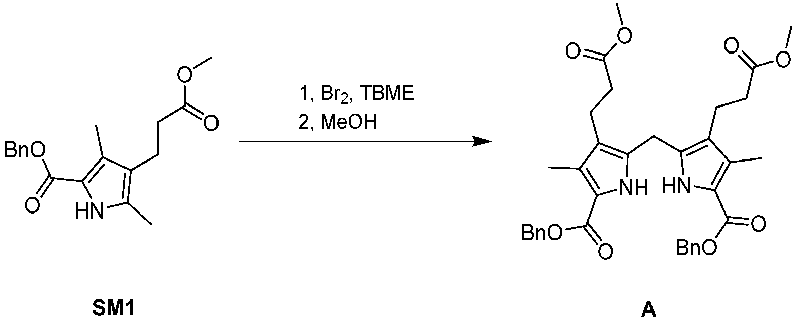

A mixture of Br₂ (53.2 g, 333 mmol, 1.4 equiv.) in MTBE (375 mL) was added dropwise to a mixture of compound SM1 (75.0 g, 238 mmol, 1.0 equiv.) in MTBE (1125 mL) at 20 °C under nitrogen condition. The mixture was stirred at 20 °C for 1 hour, followed by confirming complete reaction through TLC. The solvent was removed under reduced pressure condition. Then, methanol (546 mL) was added to the mixture. The mixture was stirred at 50 °C for 12 hours, followed by confirming complete consumption of the reactants through TLC. The mixture was cooled to 20 °C and concentrated under reduced pressure condition. After the mixture was triturated with methanol (100 mL) at 20 °C, the filtered product was washed with methanol (50 mL x 2) to yield Compound A as a gray solid (59.0 g, 95.9 mmol, yield: 81%).

¹H NMR (400 MHz, CDCl₃) δ 9.11 (s, 2H), 7.41 - 7.25 (m, 10H), 5.26 (s, 4H), 3.97 (s, 2H), 3.58 (s, 6H), 2.77 (t, *J=* 7.2 Hz, 4H), 2.52 (t, *J=* 6.8 Hz, 4H), 2.29 (s, 6H).

### (1-2) Preparation of Compound B

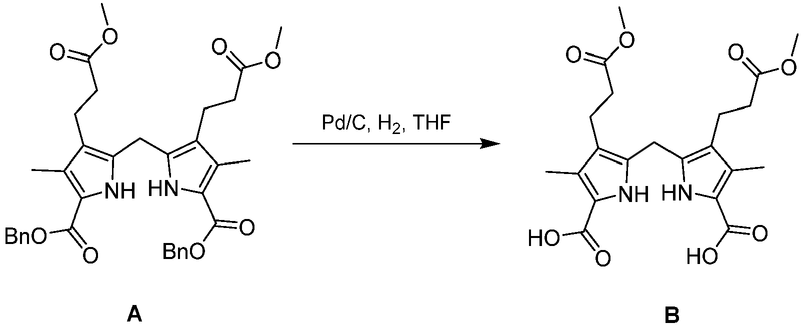

To a mixture of Compound A (50.0 g, 81.3 mmol, 1.0 equiv.) prepared above in THF (650 mL), Pd/C (5.00 g, 10 mol%) was added under nitrogen condition. The mixture was degassed under vacuum condition and filled with H₂ several times. The mixture was stirred at 20 °C for 16 hours under H₂ condition (15 psi). A mixture of Na₂CO₃ (8.62g, 81.3 mmol) and H₂O (50 mL) was added to the above mixture and stirred for 0.5 hours. The mixture was filtered and the filtrate was adjusted to pH=7 by adding acetic acid (ca. 10 mL) thereto. The precipitate was filtered and dried to yield Compound B as a pink solid (34.0 g, 78.3 mmol, yield: 96%).

¹H NMR (400 MHz, DMSO-d₆) δ 11.09 (s, 2H), 3.78 (s, 2H), 3.56 (s, 6H), 2.56 (t, *J* = 7.2 Hz, 4H), 2.16 - 2.10 (m, 10H).

### (1-3) Preparation of Compound C

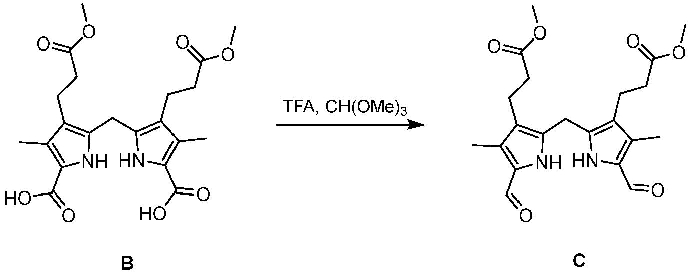

Compound B (20.0 g, 46.1 mmol, 1.0 equiv.) prepared above was added to TFA (190 mL) at 0 °C. The mixture was stirred at 0 °C for 1 hour under nitrogen condition, and trimethoxymethane (55.2 g, 520 mmol, 11.3 equiv.) was added to the mixture at 0 °C. The mixture was then stirred at 0 °C for 1 hour and monitored by LCMS. The mixture was added to 1.7 L of water and stirred for 10 minutes. The resulting precipitate was filtered and washed with 0.3 L of water. The filtered solid was triturated with ethanol (0.2 L) and ammonium hydroxide (0.4 L) at 20 °C for 30 minutes. The precipitate was filtered to give a yellow powder then washed with water (0.3 L). Methanol (0.4 L) was added to the product and refluxed for 10 minutes. After cooling the mixture to room temperature, the precipitate was filtered and washed with cold methanol (0.1 L) to yield Compound C corresponding to the compound represented by Formula 1 herein as a brown solid (12.0 g, 29.8 mmol, yield: 65%).

¹H NMR (400 MHz, CDCl₃) δ 10.19 - 10.00 (m, 2H), 9.47 (s, 2H), 4.05 (s, 2H), 3.71 (s, 6H), 2.80 (t, *J* = 7.2 Hz, 4H), 2.61 - 2.45 (m, 4H), 2.29 (s, 6H).

### Example 2: Preparation of Compound D

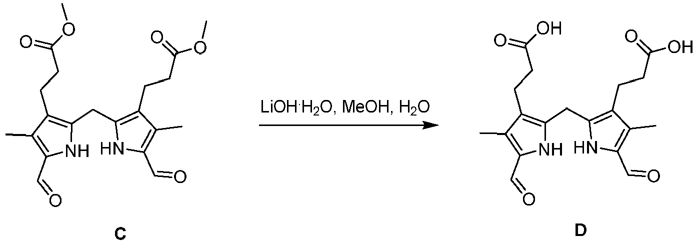

Lithium hydroxide (LiOH·H₂O) (2.75 g, 65.6 mmol, 6.6 equiv.) was added to a mixture of methanol (100 mL) and water (100 mL) as well as Compound C (4.00 g, 9.94 mmol, 1.0 equiv.) of Example 1 above. The mixture was stirred at 25 °C for 16 hours and then diluted with water (100 mL). 1M hydrochloric acid was added dropwise to the mixture to adjust the pH to 2-3. Thereafter, the precipitate was filtered and dried to yield Compound D corresponding to the compound represented by Formula 1 herein as a purple solid (3.49 g, 9.32 mmol, yield: 94%).

¹H NMR (400 MHz, CDCl₃) δ 12.03 (brs, 2H), 11.51 (s, 2H), 9.48 (s, 2H), 3.91 (s, 2H), 2.54 (overlapped with DMSO-*d*₆'s signal, 4H), 2.18 (s, 6H), 2.06 (t, *J* = 8.0 Hz, 4H).
C₁₉H₂₂N₂OS m/z [M+H]⁺ = 375

### Example 3: Preparation of Compound C1

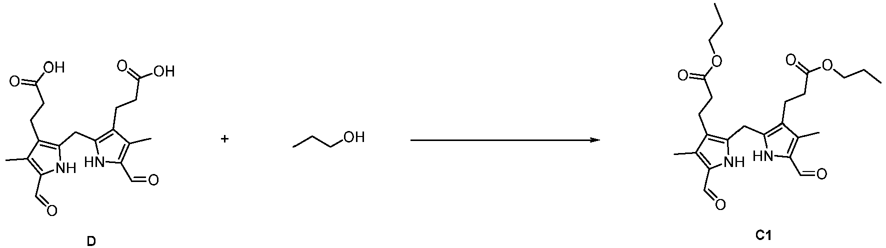

DIPEA (103.56 mg, 0.80 mmol, 3.0 equiv.), HOBt (108.28 mg, 0.80 mmol, 3.0 equiv.) and EDCI (153.61 mg, 0.80 mmol, 3.0 equiv.) were added to a mixture of Compound D (100 mg, 0.26 mmol, 1.0 equiv.) of Example 2 in DMSO (3 mL), and stirred at 25 °C for 30 minutes. 1-propanol (80.26 mg, 1.34 mmol, 5.0 equiv.) was added dropwise to the mixture and stirred for 12 hours. After adding EtOAc (140 mL) to the mixture, it was washed with water (120 mL x 5). The mixed organic layer was washed with brine (120 mL), dried over anhydrous Na₂SO₄, filtered and concentrated under reduced pressure. The obtained residue was triturated with MeOH (1 mL) and filtered under reduced pressure to yield Compound C1 corresponding to the compound represented by Formula 1 herein as a pink solid (27 mg, 0.046 mmol, yield: 21%).

¹H NMR (400 MHz, DMSO-d₆) δ 11.53 (brs, 2H), 9.47 (s, 2H), 3.91 (t, *J* = 6.8 Hz, 6H), 2.54 (overlapped with DMSO-*d*₆'s signal, 4H), 2.17 (s, 6H), 2.06 (t, *J* = 8.0 Hz, 4H), 1.58 - 1.49 (m, 4H), 0.84 (t, *J* = 7.6 Hz, 3H).

### Example 4: Preparation of Compound C2

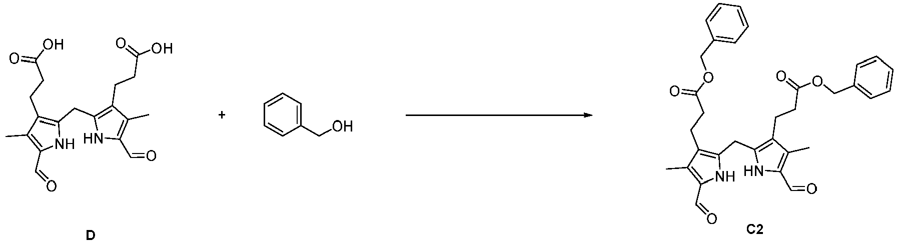

DIPEA (165.56 mg, 1.28 mmol, 3.0 equiv.), HOBt (173.24 mg, 1.28 mmol, 3.0 equiv.) and EDCI (254.78 mg, 1.28 mmol, 3.0 equiv.) were added to a mixture of Compound D (160 mg, 0.42 mmol, 1.0 equiv.) of Example 2 in DMSO (5 mL), and stirred at 25 °C for 30 minutes. After adding benzylalcohol (231.07 mg, 2.14 mmol, 5.0 equiv.) dropwise to the mixture, the mixture was stirred for 12 hours. After adding EtOAc (180 mL) to the mixture, it was washed with water (130 mL x 5). The mixed organic layer was washed with brine (130 mL), dried over anhydrous Na₂SO₄, filtered and concentrated under reduced pressure. The obtained residue was triturated with MeOH/MTBE (v/v=1/1, 10 mL) and then filtered under reduced pressure to yield Compound C2 corresponding to the compound represented by Formula 1 herein as a brown solid (110 mg, 0.198 mmol, yield: 47%).

¹H NMR (400 MHz, DMSO-*d*₆) δ 11.53 (s, 2H), 9.45 (s, 2H), 7.37 - 7.26 (m, 10H), 5.03 (s, 4H), 3.87 (s, 2H), 2.53 (overlap with DMSO-*d*₆'s signal, 4H), 2.17 - 2.14 (m, 10H).

### 2. Preparation of compound represented by Formula 2

Compounds corresponding to the compound represented by Formula 2 herein were prepared as follows (Examples 5 to 12).

### Example 5: Preparation of Compound Ga

### (5-1) Preparation of Compound Ga-2

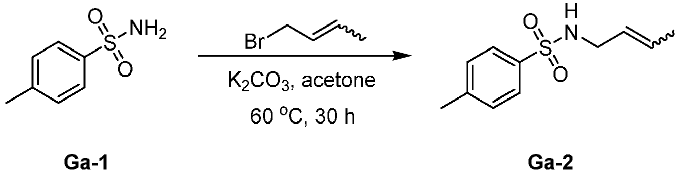

To a mixture of Compound Ga-1 (55.8 g, 326 mmol, 1.1 equiv.) prepared above in acetone (400 mL), K₂CO₃ (45.0 g, 326 mmol, 1.1 equiv.) and 1-bromobut-2-ene (40.0 g), 296 mmol, 1.0 equiv.) were added and stirred at 60 °C for 30 hours under nitrogen condition. The mixture was filtered and concentrated under reduced pressure. The residue was purified by silica gel chromatography to yield Compound Ga-2 as a yellow solid (42.4 g, yield: 64%).

¹H NMR (400 MHz, CDCl₃) δ 7.75 (d, *J*= 8.4 Hz, 2H), 7.30 (d, *J*= 8.0 Hz, 2H), 5.59 - 5.51 (m, 1H), 5.36 - 5.28 (m, 1H), 4.95 - 4.78 (m, 1H), 3.62 - 3.48 (m, 2H), 2.43 (s, 3H), 1.60 - 1.53 (m, 3H).

### (5-2) Preparation of Compound Ga-3

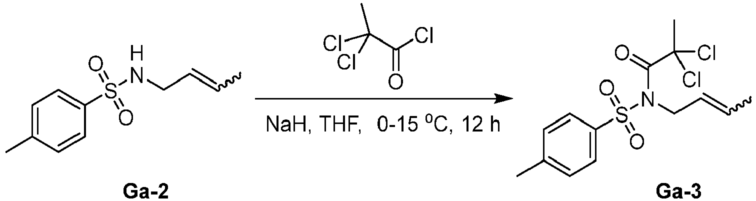

To a mixture of Compound Ga-2 (5.00 g, 22.2 mmol, 1.0 equiv.) prepared above in THF (50 mL), NaH (1.33 g, 33.3 mmol, 60% purity in mineral oil, 1.5 equiv.) was added at 0 °C. A mixture of 2,2-dichloropropanoyl chloride (9.79 g, 60.7 mmol, 2.7 equiv.) in DCM (15 mL) was added to the mixture at 0 °C and stirred at 15 °C for 12 hours. Water (100 mL) was added to this mixture and extracted with DCM (200 mL x 2). The mixed organic layer was dried over anhydrous Na₂SO₄, filtered and concentrated under reduced pressure. The residue was purified by silica gel chromatography to yield Compound Ga-3 as a yellow solid (4.47 g, 4.6 mmol, yield: 21%).

¹H NMR (400 MHz, CDCl₃) δ 7.80 (d, *J*= 8.4 Hz, 2H), 7.24 (d, *J*= 8.0 Hz, 2H), 5.90 - 5.81 (m, 1H), 5.60 - 5.50 (m, 1H), 4.99 - 4.86 (m, 2H), 2.37 (s, 3H), 2.13 (s, 3H), 1.75 - 1.69 (m, 3H).

### (5-3) Preparation of Compound Ga-4

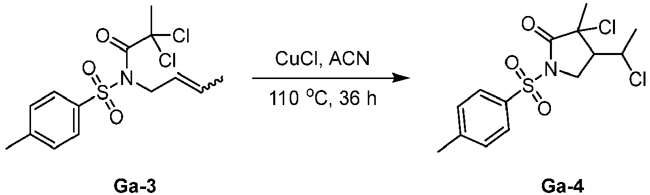

CuCl (1.05 g, 10.6 mmol, 0.4 equiv.) was added to a mixture of Compound Ga-3 (9.29 g, 26.5 mmol, 1.0 equiv.) prepared above in ACN (40 mL) and stirred at 110 °C for 36 hours under nitrogen condition. This mixture was concentrated under reduced pressure condition. The residue was purified by silica gel chromatography to yield Compound Ga-4 in the form of yellow oil (8.50 g, 24.1 mmol, yield: 91%).
C₁₄H₁₇Cl₂NO₃S m/z [M+H]⁺ = 350.0

### (5-4) Preparation of Compound Ga-5

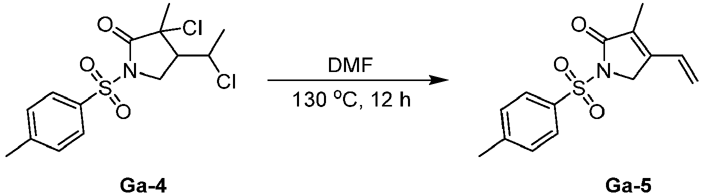

A mixture of Compound Ga-4 (8.50 g, 24.3 mmol, 1.0 equiv.) prepared above in DMF (90 mL) was stirred at 130 °C for 12 hours under nitrogen condition. EtOAc (250 mL) was added to the mixture and washed with water (50 mL x 4). The mixed organic layer was washed with brine (50 mL X2), dried over anhydrous Na₂SO₄, filtered and concentrated under reduced pressure condition. The residue was purified by silica gel chromatography to yield Compound Ga-5 as a yellow solid (4.70 g, 17.0 mmol, yield: 70%).

¹H NMR (400 MHz, CDCl₃) δ 7.90 (d, *J* = 8.4 Hz, 2H), 7.26 (d, J = 8.0 Hz, 2H), 6.62 (dd, *J* = 17.6, 11.2 Hz, 1H), 5.51 (d, *J* = 18.0 Hz, 1H), 5.43 (d, *J* = 10.8 Hz, 1H), 4.41 (d, *J* = 1.2 Hz, 2H), 2.36 (s, 3H), 1.76 (s, 3H).

### (5-5) Preparation of Compound Ga

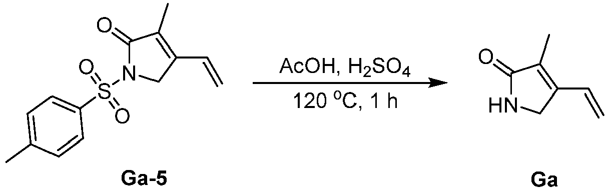

A mixture of Compound Ga-5 (5.00 g, 18.0 mmol, 1.0 equiv.) prepared above with acetic acid (9.46 g, 158 mmol, 8.7 equiv.) and H₂SO₄ (16.6 g, 162 mmol, 9.0 equiv.) was stirred at 120 °C for 1 hour. To this mixture, 10% Na₂CO₃ aqueous solution (300 mL) was added, followed by extraction with DCM (100 mL x 5). The mixed organic layer was dried over anhydrous Na₂SO₄, dried and concentrated under reduced pressure condition thus to yield Compound Ga corresponding to the compound represented by Formula 2 herein as a yellow solid (1.18 g, 9.58 mmol, yield: 53%).

¹H NMR (400 MHz, CDCl₃) δ 6.79 (brs, 1H), 6.73 (dd, *J=* 17.6, 11.2 Hz, 1H), 5.45 (d, *J=* 17.6 Hz, 1H), 5.37 (d, *J=* 10.8 Hz, 1H), 4.06 (s, 2H), 1.92 (s, 3H).
C₇H₉NO m/z [M+H]⁺ = 124

### Example 6: Preparation of Compound Gb

### (6-1) Preparation of Compound Gb-1

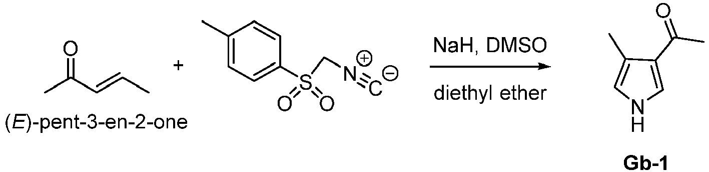

To a mixture of NaH (135 g, 3.39 mmol, 1.68 equiv.) in diethyl ether (5.5 mL), a mixture of (E)-pent-3-en-2-one (0.2 g, 2.02 mmol, 1.0 equiv.) and TosMIC (0.57 g, 2.91 mmol, 1.44 equiv.) in diethyl ether/DMSO (10 mL/5 mL) was added dropwise. The mixture was stirred at 25 °C for 1 hour 30 minutes and the reaction was terminated by adding water (30 mL). After extraction with diethyl ether (90 mL), the mixture was dried over anhydrous Na₂SO₄, filtered and concentrated under reduced pressure condition. The residue was purified by silica gel chromatography to yield Compound Gb-1 (140 mg, 1.13 mmol, yield: 56%).

¹H NMR (400 MHz, CDCl₃) δ 8.74 (brs, 1H), 7.36 (s, 1H), 6.55 (s, 1H), 2.40 (s, 3H), 2.31 (s, 3H).

### (6-2) Preparation of Compound Gb

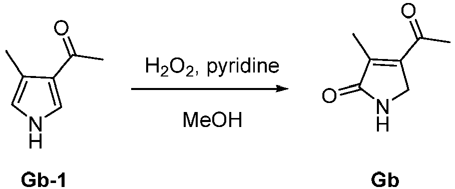

To a mixture of Compound Gb-1 (131.4 mg, 1.07 mmol, 1.0 equiv.) prepared above in methanol (5 mL), 30% H₂O₂ (0.61 mL, 5.35 mmol, 5.0 equiv.) and pyridine (0.21 mL, 2.67 mmol, 2.5 equiv.) were added and stirred at 60 °C for 16 hours. To the mixture, 30% H₂O₂ (0.61 mL, 5.35 mmol, 5.0 equiv.) and pyridine (0.21 mL, 2.67 mmol, 2.5 equiv.) were further added and stirred at 60 °C for 12 hours. After cooling the mixture to 25 °C, the solvent was removed under reduced pressure condition, and the obtained residue was purified by silica gel chromatography to yield Compound Gb corresponding to the compound represented by Formula 2 herein (14 mg, 0.10 mmol, yield: 9%).

¹H NMR (400 MHz, CDCl₃) δ 7.59 (brs, 1 H), 4.12 (s, 2H), 2.45 (s, 3H), 2.21 (s, 3H).

### Example 7: Preparation of Compound Gc

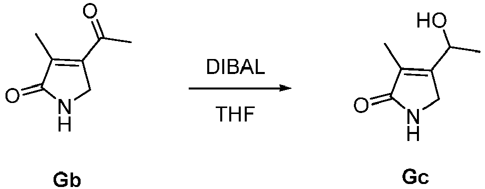

1.0M DIBAL in THF (0.36 mL, 0.36 mmol, 2.2 equiv.) was added dropwise to a mixture of Compound Gb (23.2 mg, 0.17 mmol, 1.0 equiv.) prepared above in THF (1.6 mL) at -10 °C and stirred for 1 hour. The reaction was terminated with NH₄Cl aqueous solution (5.0 mL), extracted with DCM (15 mL x 5), dried over anhydrous Na₂SO₄ and concentrated under reduced pressure condition to yield Compound Gc in the form of yellow oil (6.9 mg, 0.049 mmol, yield: 29.4%).
C₇H₁₁NO₂ m/z [M+H]⁺ = 142

### Example 8: Preparation of Compound Gd

### (8-1) Preparation of Compound Gd-1

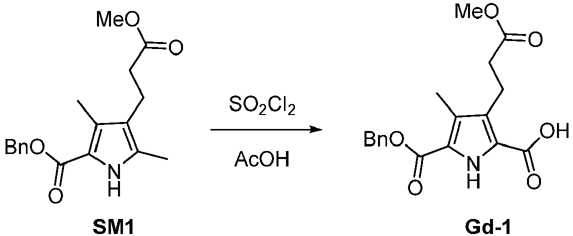

A mixture of compound SM1 (5 g, 15.85 mmol, 1.0 equiv.) in acetic acid (120 mL) was stirred at 25 °C for 30 minutes, then SO₂Cl₂ (1M in DCM, 31.7 mmol, 31.7 mL, 2.0 equiv.) was added thereto and stirred at 25 °C for 1 hour. SO₂Cl₂ (1M in DCM, 31.7 mmol, 31.7 mL, 2.0 equiv.) was further added and stirred at 25°C for 1 hour. DCM (300 mL) was added, followed by washing with water (400 mL x 8). The mixed organic layer was dried over anhydrous Na₂SO₄, filtered under reduced pressure condition and concentrated to yield Compound Gd-1 in the form of brown oil (5.9 g, yield: >99%).

¹H NMR (400 MHz, CDCl₃) δ 9.56 (s, 1H), 7.44 - 7.35 (m, 5H), 5.34 (s, 2H), 3.67 (s, 3H), 3.06 (t, *J* = 8.1 Hz, 2H), 2.55 (t, *J* = 8.0 Hz, 2H), 2.30 (s, 3H).

### (8-2) Preparation of Compound Gd-2

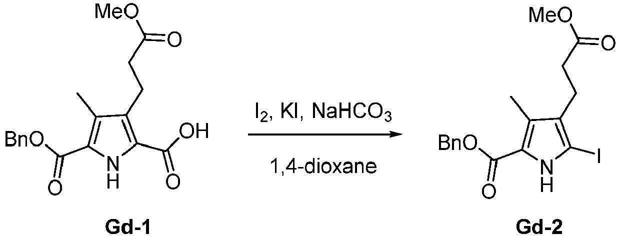

To a mixture of Compound Gd-1 (5.9 g, 17.08 mmol, 1.0 equiv.) prepared above in 1,4-dioxane (250 mL), NaHCO₃ aqueous solution (4.3 g, 51.24 mmol, 3.0 equiv., 250 mL of water) was added and then stirred at 25 °C for 30 minutes. I₂ (4.34 g, 17.08 mmol, 1.0 equiv.) and KI (7.1 g, 42.7 mmol, 2.5 equiv.) were added to the mixture and stirred at 60 °C for 3 hours. After cooling to 25 °C, NaHSO₃ aqueous solution (100 mL) was added, followed by extraction with DCM (250 mL). The mixed organic layer was washed with brine (200 mL x 2) and dried over anhydrous Na₂SO₄. The mixture was filtered and concentrated under reduced pressure condition to yield Compound Gd-2 in the form of brown oil (6.1 g, 14.2 mmol, yield: 83%).

¹H NMR (400 MHz, CDCl₃) δ 9.35 (s, 1H), 7.42 - 7.32 (m, 5H), 5.31 (s, 2H), 3.67 (s, 3H), 2.70 (t, *J* = 7.5 Hz, 2H), 2.56 (t, *J* = 7.4 Hz, 2H), 2.32 (s, 3H).

### (8-3) Preparation of Compound Gd-3

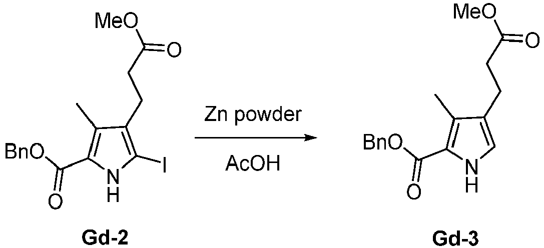

To a mixture of Compound Gd-2 (6.1 g, 14.28 mmol, 1.0 equiv.) prepared above in AcOH (142 mL), Zn (6.06 g, 92.69 mmol, 6.5 equiv.) was added and stirred at 120 °C for 3 hours. Zn was removed under reduced pressure condition, and DCM (300 mL) was added to the residue, followed by washing with water (400 mL) several times. The mixed organic layer was dried over anhydrous Na₂SO₄, filtered and concentrated under reduced pressure condition to yield Compound Gd-3 in the form of yellow oil (4.1 g, 13.60 mmol, yield: 95%).

¹H NMR (400 MHz, CDCl₃) δ 8.74 (s, 1H), 7.41 - 7.31 (m, 5H), 5.29 (s, 2H), 3.66 (s, 3H), 2.75 (t, *J* = 7.7 Hz, 2H), 2.54 (t, *J* = 7.7 Hz, 2H), 2.30 (s, 3H).

### (8-4) Preparation of Compound Gd-4

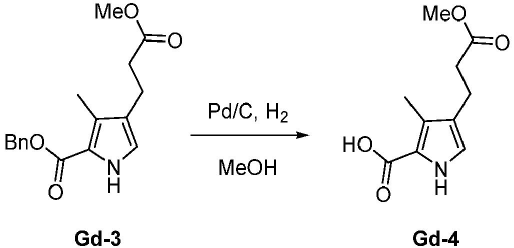

To a mixture of Compound Gd-3 (4.05 g, 13.43 mmol, 1.0 equiv.) prepared above in methanol (500 mL), Pd/C (3.16 g, 10 mol%) was added under nitrogen condition. The mixture was degassed under vacuum condition, filled with H₂ several times, and then stirred at 25 °C for 30 minutes. Pd/C was removed under reduced pressure, and the residue was concentrated to yield Compound Gd-4 as a pink solid (900 mg, 4.25 mmol, yield: 31%).

¹H NMR (500 MHz, CDCl₃) δ 8.94 (s, 1H), 6.76 (d, *J=* 3.0 Hz, 1H), 3.67 (s, 3H), 2.76 (t, *J* = 7.6 Hz, 2H), 2.55 (t, *J* = 7.6 Hz, 2H), 2.32 (s, 3H).

### (8-5) Preparation of Compound Gd-5

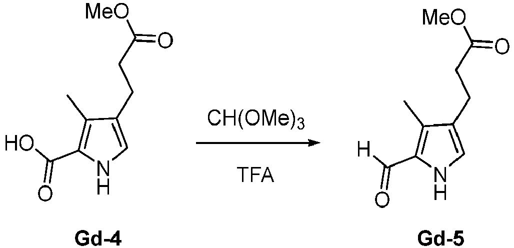

To Compound Gd-4 (299 mg, 1.41 mmol, 1.0 equiv.) prepared above, CH(OMe)₃ (0.88 mL, 7.92 mmol, 5.6 equiv.) was added at 0 °C. Then, TFA (2.4 mL) was added dropwise to the mixture at 0 °C and stirred for 1 hour 30 minutes. The mixture was neutralized with NaHCO₃ aqueous solution (30 mL) at 0 °C and then extracted with DCM (50 mL). The mixed organic layer was washed with water (40 mL x 2), dried over anhydrous Na₂SO₄, filtered and concentrated under reduced pressure condition. The residue was purified by silica gel chromatography to yield Compound Gd-5 (90.8 mg, 0.46 mmol, yield: 32%).

¹H NMR (400 MHz, CDCl₃) δ 9.52 (s, 1H), 6.88 (s, 1H), 3.62 (s, 3H), 2.70 (t, *J* = 7.8 Hz, 2H), 2.51 (t, *J* = 7.8 Hz, 2H), 2.25 (s, 3H).

### (8-6) Preparation of Compound Gd-6

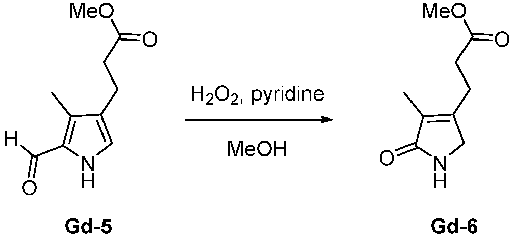

To a mixture of Compound Gd-5 (226 mg, 1.15 mmol, 1.0 equiv.) prepared above in methanol (5.5 mL), pyridine (0.24 mL, 2.89 mmol, 2.5 equiv.) and 30% H₂O₂ (0.65 mL, 5.79 mmol, 5.0 equiv.) were added and stirred at 60 °C for 16 hours. Thereafter, the solution was concentrated under reduced pressure and high temperature, and the residue was purified by silica gel chromatography to yield Compound Gd-6 (122 mg, 0.66 mmol, yield: 57%).

¹H NMR (400 MHz, CDCl₃) δ 7.06 (brs, 1H), 6.88 (s, 1H), 3.8 (d, *J=* 1.3 Hz, 2H), 3.67 (s, 3H), 2.69 (t, *J* = 7.5 Hz, 2H), 2.50 (t, *J* = 7.4 Hz, 2H), 1.80 (s, 3H).

### (8-7) Preparation of Compound Gd-7

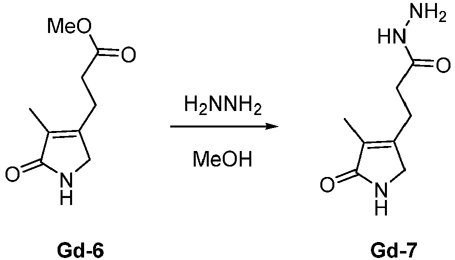

To a mixture of Compound Gd-6 (56.5 mg, 0.31 mmol, 1.0 equiv.) prepared above in methanol (3 mL), hydrazine hydrate (0.38 mL, 6.78 mmol, 22.0 equiv.) was added and refluxed for 16 hours. It was confirmed by LCMS that Compound Gd-7 was produced.
C₈H₁₃N₃O₂ m/z [M+H]⁺ = 184

### (8-8) Preparation of Compound Gd

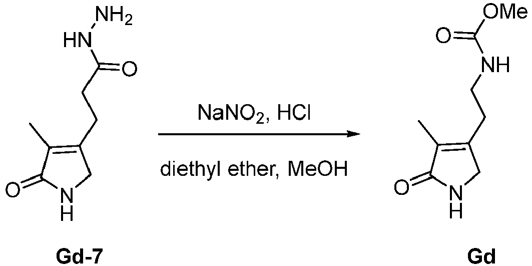

After lowering the temperature of the Compound Gd-7 (58.6 mg, 0.32 mmol, 1.0 equiv.) prepared above to 0 °C, 5N HCl aqueous solution (3.27 mmol, 10.0 equiv.) was added thereto. 0.5M NaNO₂ aqueous solution (0.36 mmol, 1.1 equiv.) was added dropwise to the mixture at 0 °C and stirred for 20 minutes. After extraction with diethyl ether several times, the mixed organic layer was dried over anhydrous Na₂SO₄ and concentrated under reduced pressure condition, leaving only 1/10 of the organic layer. Then, methanol (30 mL) was added and refluxed. The mixture was concentrated under reduced pressure condition, and the residue was purified by silica gel chromatography to yield Compound Gd corresponding to the compound represented by Formula 2 herein (5.9 mg, 0.029 mmol, yield: 9%).

¹H NMR (500 MHz, CDCl₃) δ 6.32 (brs, 1H), 4.84 (brs, 1H), 3.87 (s, 2H), 3.66 (s, 3H), 3.39 - 3.34 (m, 2H), 2.60 (t, *J* = 6.7 Hz, 2H), 1.81 (s, 3H).

### Example 9: Preparation of Compound Ge-7

### (9-2) Preparation of Compound Ge-3

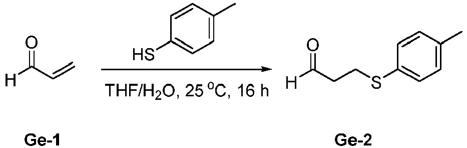

After adding Ge-1 (20.1 g, 358 mmol, 1.0 equiv.) dropwise to a mixture of 4-methylbenzenethiol (36.9 g, 297 mmol, 0.83 equiv.) in THF (300 mL) and water (150 mL) at 0 °C, it was stirred at 25 °C for 16 hours under nitrogen condition. To the mixture, NaHCO₃ aqueous solution (200 mL) was added, followed by extraction with EtOAc (200 mL x 2). The mixed organic layer was washed with brine (50 mL x 2), dried over anhydrous Na₂SO₄, filtered and concentrated under reduced pressure condition to yield Compound Ge-2 in the form of yellow oil (59.4 g, 330 mmol, yield: 92 %).

¹H NMR (400 MHz, CDCl₃) δ 9.74 (s, 1H), 7.27 (d, *J*= 8.0 Hz, 2H), 7.11 (d, *J*= 7.6 Hz, 2H), 3.13 (t, *J=* 7.2 Hz, 2H), 2.75 - 2.71 (m, 2H), 2.32 (s, 3H).

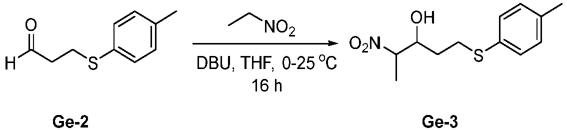

To a mixture of Compound Ge-2 (58 g, 322 mmol, 1.0 equiv.) and DBU (4.90 g, 32.2 mmol, 0.1 equiv.) in THF (400 mL), a mixture of 1-nitroethane (24.0 g, 322 mmol, 1.0 equiv.) in THF (50 mL) was added at 0 °C and stirred at 25 °C for 16 hours. The mixture was diluted with water (200 mL) then extracted with EtOAc (400 mL x 2). The mixed organic layer was washed with brine (200 mL), dried over anhydrous Na₂SO₄, filtered and concentrated under reduced pressure condition. The residue was purified by silica gel chromatography to yield Compound Ge-3 in the form of yellow oil (51.7 g, 202 mmol, yield: 63%).

¹H NMR (400 MHz, CDCl₃) δ 7.28 (d, *J*= 7.6 Hz, 2H), 7.13 (d, *J*= 8.0 Hz, 2H), 4.54 - 4.46 (m, 1H), 4.15 - 4.12 (m, 1H), 3.15 - 3.08 (m, 1H), 3.03 - 2.96 (m, 1H), 2.33 (s, 3H), 1.80 - 1.64 (m, 2H), 1.53 (t, *J* = 8.0 Hz, 3H).

### (9-3) Preparation of Compound Ge-4

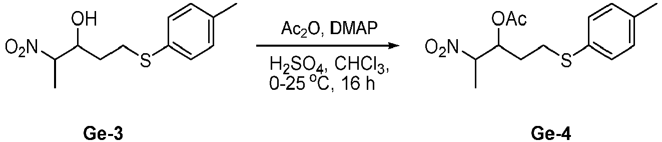

Acetic anhydride (31.0 g, 304 mmol, 1.5 equiv.) was added slowly to a mixture of Compound Ge-3 (51.7 g, 202 mmol, 1.0 equiv.) prepared above and H₂SO₄ (199 mg, 2.02 mmol, 0.01 equiv.) in chloroform (500 mL) at 0 °C and stirred at 25 °C for 16 hours. The reaction was terminated with NaHCO₃ aqueous solution (100 mL) and then extracted with DCM (50 mL x 4). The mixed organic layer was washed with brine (50 mL x 2), dried over anhydrous Na₂SO₄, filtered and concentrated under reduced pressure condition to yield Compound Ge-4 in the form of brown oil (65.5 g, yield: >99%).

¹H NMR (400 MHz, CDCl₃) δ 7.27 (d, *J* = 8.4 Hz, 2H), 7.12 (d, *J* = 8.0 Hz, 2H), 5.45 - 5.40 (m, 1H), 4.75 - 4.66 (m, 1H), 2.98 - 2.78 (m, 2H), 2.33 (s, 3H), 2.07 (d, *J* = 8.8 Hz, 3H), 1.99 - 1.80 (m, 2H), 1.49 (dd, *J=* 6.8, 2.0 Hz, 3H).

### (9-4) Preparation of Compound Ge-5

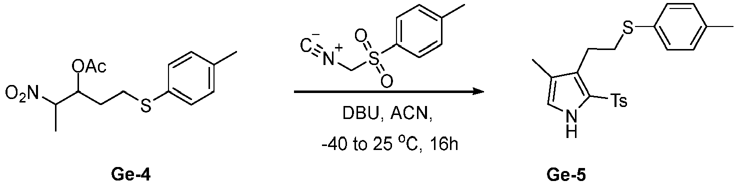

To a mixture of Compound Ge-4 (3.61 g, 18.5 mmol, 1.0 equiv.) prepared above and DBU (5.63 g, 37.0 mmol, 2.0 equiv.) in ACN (50 mL), a mixture of TosMIC (5.00 g, 16.8 mmol, 0.9 equiv.) in ACN (10 mL) was added dropwise at -40 °C in a nitrogen environment, and the mixture was stirred at 25 °C for 16 hours. The mixture was diluted with water (100 mL) and then extracted with EtOAc (100 mL x 2). The mixed organic layer was washed with brine (100 mL), dried over anhydrous Na₂SO₄, filtered and concentrated under reduced pressure condition to obtain a residue. The residue was purified by silica gel chromatography to yield Compound Ge-5 in the form of red oil (3.47 g, 9.00 mmol, yield: 53%).

¹H NMR (400 MHz, CDCl₃) δ 8.99 (s, 1H), 7.65 (d, *J=* 8.0 Hz, 2H), 7.31 (d, *J* = 8.0 Hz, 2H), 7.21 (d, *J* = 8.0 Hz, 2H), 7.14 (d, *J* = 8.0 Hz, 2H), 6.69 (d, *J* = 2.0 Hz, 1H), 2.87 - 2.81 (m, 4H), 2.37 (s, 3H), 2.35 (s, 3H), 1.93 (s, 3H).

### (9-5) Preparation of Compound Ge-6

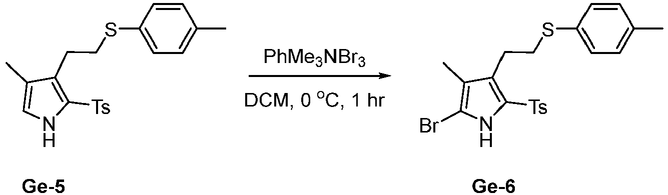

PhMe₃NBr₃ (3.31 g, 8.56 mmol, 1.1 equiv.) was added to a mixture of Compound Ge-5 (3 g, 7.78 mmol, 1.0 equiv.) prepared above in DCM (78 mL) and stirred at 0 °C for 1 hour. Then, NaHSO₃ aqueous solution (50 mL) was added to the mixture to terminate the reaction, followed by extraction with DCM (80 mL) and washing the organic layer with water (50 mL). The mixed organic layer was dried over anhydrous Na₂SO₄, filtered and concentrated under reduced pressure condition to yield Compound Ge-6 (3.25 g, 7.00 mmol, yield: 90%).

¹H NMR (400 MHz, CDCl₃) δ 8.92 (brs, 1H), 7.64 (d, *J* = 8.3 Hz, 2H), 7.29 (d, *J* = 8.1 Hz, 2H), 7.22 (d, *J* = 8.0 Hz, 2H), 7.13 (d, *J* = 8.0 Hz, 2H), 2.86 - 2.77 (m, 4H), 2.37 (s, 3H), 2.34 (s, 3H), 1.85 (s, 3H).

### (9-6) Preparation of Compound Ge-7

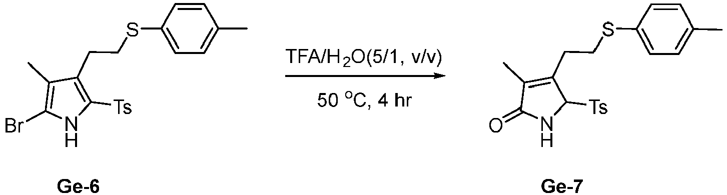

TFA/H₂O (v/v=5/1, 6.46 mL/1.29 mL) was added to Compound Ge-6 (600 mg, 1.29 mmol, 1.0 equiv.) prepared above, and the mixture was stirred at 50 °C for 4 hours. The reaction was terminated by neutralizing the mixture with Na₂CO₃ aqueous solution. The organic layer was extracted by adding DCM (50 mL) and water (40 mL). The mixed organic layer was dried over anhydrous Na₂SO₄, filtered and concentrated under reduced pressure condition. The residue was purified by silica gel chromatography to yield Compound Ge-7 corresponding to the compound represented by Formula 2 herein (250 mg, 0.62 mmol, yield: 48%).

¹H NMR (400 MHz, CDCl₃) δ 7.54 (d, *J* = 6.5 Hz, 1H), 7.20 - 7.17 (m, 4H), 7.04 (d, *J* = 6.3 Hz, 2H), 3.21 - 3.16 (m, 1H), 3.07 - 3.02 (m, 1H), 2.99 - 2.94 (m, 1H), 2.73 - 2.67 (m, 1H), 2.37 (s, 3H), 2.32 (s, 3H), 2.24 (s, 3H), 1.42 (s, 3H).

### Example 10: Preparation of Compound Ge

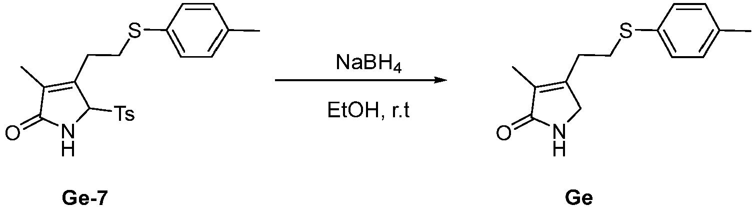

NaBH₄ (5 mg, 0.13 mmol, 1.1 equiv.) was added to a mixture of Compound Ge-7 (47.2 mg, 0.12 mmol, 1.0 equiv.) prepared above in EtOH (5 mL) and stirred at 25 °C for 30 minutes. To the mixture, NH₄Cl aqueous solution (5 mL) was added and extracted with DCM (20 mL). The mixed organic layer was dried over anhydrous Na₂SO₄, filtered and concentrated under reduced pressure condition. The residue was purified by silica gel chromatography to yield Compound Ge corresponding to the compound represented by Formula 2 herein (29 mg, 0.057 mmol, yield: 49%).

¹H NMR (500 MHz, CDCl₃) δ 7.19 (d, *J* = 8.1 Hz, 2H), 7.05 (d, *J=* 8.0 Hz, 2H), 3.78 (s, 2H), 2.93 (d, *J=* 7.4 Hz, 2H), 2.58 (d, *J=* 7.3 Hz, 2H), 2.26 (s, 3H), 1.69 (s, 3H).

### Example 11: Preparation of Compound Gf-7

### (11-1) Preparation of Compound Gf-1

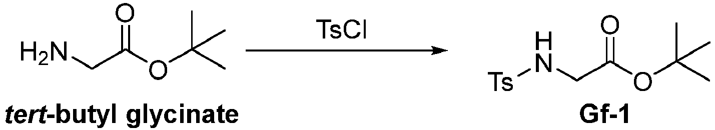

TsCl (1.25 g, 6.56 mmol, 1.1 equiv.) was added to a mixture of tert-butyl glycinate (1 g, 5.96 mmol, 1.0 equiv.) in 1M NaHCO₃ aqueous solution (2.5 equiv.) and stirred at 25 °C for 24 hours. The reaction mixture was extracted with EtOAc (50 mL x 3). The mixed organic layer was washed with brine (50 mL), dried over anhydrous Na₂SO₄, filtered and concentrated under reduced pressure condition. The residue was purified by silica gel chromatography to yield Compound Gf-1 (1.45 g, 4.94 mmol, yield: 83%).
C₁₃H₁₉NO₄S m/z [M+2H-C₄H₉^{•}]⁺ = 230

### (11-2) Preparation of Compound Gf-2

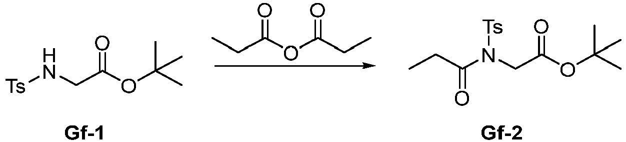

1.3M LiHMDS in THF (4 mL, 5.23 mmol, 1.15 equiv.) was added dropwise to a mixture of Compound Gf-1 (1.3 g, 4.55 mmol, 1.0 equiv.) prepared above in THF (13 mL) at -78 °C and stirred for 1 hour. At -78 °C, propionic anhydride (0.75 mL, 5.92 mmol, 1.3 equiv.) was added and stirred for 1 hour, followed by further stirring at 25 °C for additional 1 hour. The reaction was terminated with NH₄Cl aqueous solution (30 mL) and extracted with DCM (40 mL). The mixed organic layer was dried over anhydrous Na₂SO₄, filtered and concentrated under reduced pressure condition. The residue was purified by silica gel chromatography to yield Compound Gf-2 (1.4 g, 4.23 mmol, yield: 93%).

¹H NMR (400 MHz, CDCl₃) δ 7.88 (d, *J* = 8.3 Hz, 2H), 7.33 (d, *J* = 8.0 Hz, 2H), 4.50 (s, 2H), 2.56 (q, *J* = 7.2 Hz, 2H), 2.43 (s, 3H), 1.44 (s, 9H), 1.02 (t, *J* = 7.2 Hz, 3H).

### (11-3) Preparation of Compound Gf-3

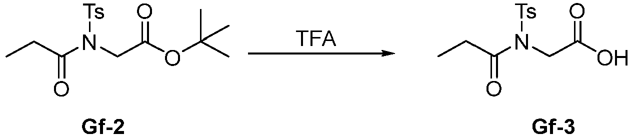

To a mixture of Compound Gf-2 (500 mg, 1.46 mmol, 1.0 equiv.) prepared above in DCM (5 mL), TFA (1.5 mL) was added at 0 °C and stirred at 25 °C for 2 hours. The solid obtained by concentrating under reduced pressure condition was filtered to yield Compound Gf-3 (384 mg, 1.34 mmol, yield: 92%).

¹H NMR (500 MHz, DMSO-d₆) δ 13.18 (brs, 1H), 7.88 (d, *J* = 8.1 Hz, 2H), 7.43 (d, *J* = 8.0 Hz, 2H), 4.57 (s, 2H), 2.47 (q, *J=* 7.2 Hz, 2H), 2.40 (s, 3H), 0.87 (t, *J=* 7.1 Hz, 3H).

### (11-4) Preparation of Compound Gf-4

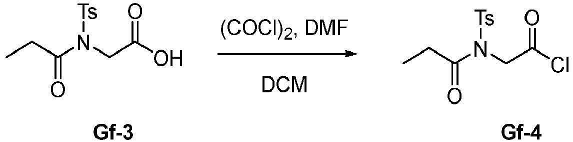

To a mixture of Compound Gf-3 (100 mg, 0.35 mmol, 1.0 equiv.) in DCM (2 mL), (COCl)₂ (36 µL, 0.42 mmol, 1.2 equiv.) and DMF (2 drops) were added and stirred at 0 °C for 1 hour under nitrogen condition. Then, it was concentrated under reduced pressure condition to yield Compound Gf-4.

(In order to confirm the reactive Gf-4 through LCMS, methanol was added to change it to an ester form (C₁₃H₁₇NO₅S), followed by confirmation).
C₁₃H₁₇NO₅S m/z [M+H]⁺ = 300

### (11-5) Preparation of Compound Gf-5

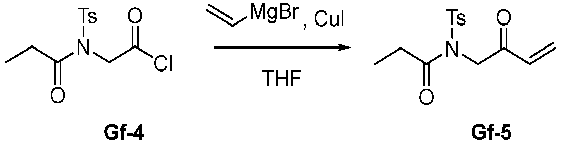

To a mixture of Compound Gf-4 (1.7 mmol, 1.0 equiv.) in THF (10 mL), CuI (65 mg, 0.34 mmol, 0.2 equiv.) was added at -20 °C. Then, 1.0 M vinyl MgBr in THF solution (3.4 mmol, 3.5 mL, 2.0 equiv.) was added dropwise at -20 °C, followed by stirring at 25 °C for 16 hours. The reaction was terminated with aqueous NH₄Cl (10 mL) and extracted with EtOAc (30 mL). The mixed organic layer was washed with brine (20 mL), dried over anhydrous Na₂SO₄, filtered and concentrated under reduced pressure condition. The residue was purified by silica gel chromatography to yield Compound Gf-5 (40 mg, 0.56 mmol, 2 steps yield: 38%).

¹H NMR (500 MHz, CDCl₃) δ 7.90 (d, *J*= 8.0 Hz, 2H), 7.32 (d, *J* = 7.9 Hz, 2H), 6.43 (dd, *J* = 17.0, 10.9 Hz, 1H), 5.54 (d, *J* = 17.1 Hz, 1H), 5.46 (d, *J* = 10.8 Hz, 1H), 4.75 (s, 2H), 2.46 (t, *J* = 7.1 Hz, 2H), 0.92 (t, *J* = 7.1 Hz, 3H).

### (11-6) Preparation of Compound Gf-6

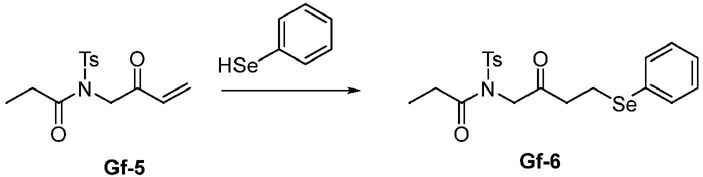

Benzeneselenol (71.5 µL, 0.65 mmol, 5.0 equiv.) was added to a mixture of Compound Gf-5 (40 mg, 0.13 mmol, 1.0 equiv.) prepared above in pyridine (1.5 mL), and the mixture was stirred in a microwave at 150°C for 10 minutes. After removing pyridine under reduced pressure and high temperature conditions, NH₄Cl aqueous solution (20 mL) was added, followed by extraction with DCM (20 mL). The mixed organic layer was dried over anhydrous Na₂SO₄, filtered and concentrated under reduced pressure condition to yield Compound Gf-6.
C₂₀H₂₃NO₄SSe m/z [M+H]⁺ = 454

### (11-7) Preparation of Compound Gf-7

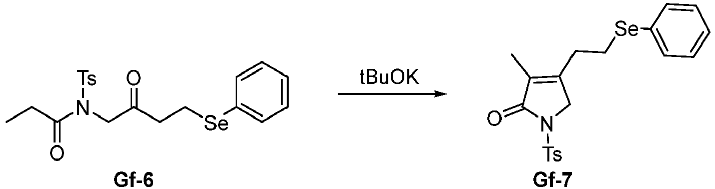

To a mixture of Compound Gf-6 (30 mg, 0.066 mmol, 1.0 equiv.) in THF (1 mL), 1M tBuOK in THF solution (0.13 mL, 0.13 mmol, 2.0 equiv.) was added dropwise at -10°C, and stirred for 30 minutes. The reacted solution was diluted with water (15 mL) and extracted with DCM (20 mL). The mixed organic layer was washed with brine (15 mL x 2), dried over anhydrous Na₂SO₄, filtered and concentrated under reduced pressure condition. The residue was purified by silica gel chromatography to yield Compound Gf-7 corresponding to the compound represented by Formula 2 herein (10 mg, 0.023 mmol, yield: 34%).

¹H NMR (500 MHz, CDCl₃) δ 7.94 (d, J = 10.4 Hz, 2H), 7.48 - 7.46 (m, 2H), 7.32 (d, J = 10.1 Hz, 2H), 7.27 - 7.25 (m, 3H), 4.27 (d, *J* = 2.1 Hz, 2H), 2.98 (t, *J* = 9.3 Hz, 2H), 2.74 (t, *J* = 9.2 Hz, 2H), 2.42 (s, 3H), 1.63 (s, 3H).

### Example 12: Preparation of Compound Gf

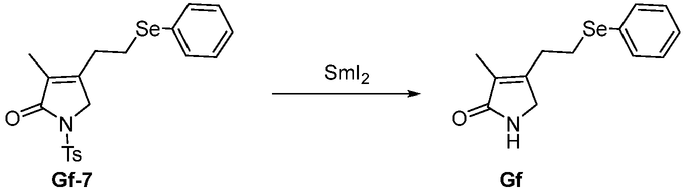

To a mixture of Compound Gf-7 (148.6 mg, 0.34 mmol, 1.0 equiv.) prepared above in THF (7 mL), 0.1M SmI₂ in THF solution (17.1 mL, 5.0 equiv.) was added dropwise at 0 °C under nitrogen condition. After stirring at 25 °C for 10 minutes, the reaction was terminated with NaHCO₃ aqueous solution (10 mL). It was extracted with EtOAc (50 mL) and washed with water (30 mL x 2). The mixed organic layer was dried over anhydrous Na₂SO₄, filtered and concentrated under reduced pressure condition. The residue was purified by silica gel chromatography to yield Compound Gf corresponding to the compound represented by Formula 2 herein (62.3 mg, 0.22 mmol, yield: 65%).

¹H NMR (400 MHz, CDCl₃) δ 7.51 - 7.48 (m, 2H), 7.28 - 7.26 (m, 2H), 3.83 (s, 2H), 3.00 (t, *J* = 7.4 Hz, 2H), 2.75 (t, *J* = 7.5 Hz, 2H), 1.75 (s, 3H).

### 3. Preparation of compound represented by Formula 3

The compound represented by Formula 1 and the compound represented by Formula 2 were coupled to prepare the compound represented by Formula 3 as follows (Examples 13 to 44).

### 3.1. Preparation of Compound F-13a by coupling of Compound D and Compound Ga

Compound F-13a corresponding to the compound represented by Formula 3 was prepared by coupling the Compound D of Example 2 with the Compound Ga of Example 5.

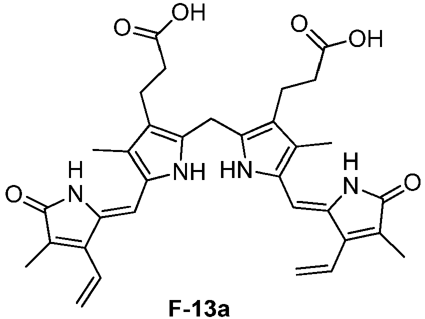

### Example 13

To a mixture of Compound D (1.34 g, 3.57 mmol, 1.0 equiv.) and Compound Ga (1.10 g, 8.93 mmol, 2.5 equiv.) in 1,4-dioxane (15 mL), piperidine (2.81 g, 28.3 mmol, 8.0 equiv.) was added and stirred at 100 °C for 12 hours. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a was obtained as a red solid by filtration under reduced pressure condition (939 mg, 1.61 mmol, yield: 45%).

¹H NMR (400 MHz, DMSO-d₆) δ 11.91 (brs, 2H), 10.44 (s, 2H), 10.03 (s, 2H), 6.82 (dd, *J* = 17.6, 12.0 Hz, 2H), 6.09 (s, 2H), 5.64 (d, *J* = 10.8 Hz, 2H), 5.61 (dd, *J* = 8.8, 1.6 Hz, 2H), 3.98 (s, 2H), 2.44 - 2.40 (m, 4H), 2.00 (s, 6H), 1.96 - 1.94 (m, 4H), 1.92 (s, 6H).

¹³C NMR (400 MHz, DMSO-d₆) δ 174.52, 171.83, 140.87, 131.35, 127.87, 123.92, 123.68, 122.57, 122.54, 120.02, 99,66, 34.82, 24.01, 19.77, 9.98, 9.63.
C₃₃H₃₆N₄O₆ m/z [M+H]⁺ = 585

### Example 14: Preparation of Compound F-13a

Azepane (8.0 equiv.) and Compound Ga (2.5 equiv.) were added to a mixture of Compound D (1.0 equiv.) and 1,4-dioxane, and stirred at 100 °C for 16 hours under nitrogen condition. A reaction conversion ratio was measured by liquid chromatography mass spectrometry (LCMS), and the reaction conversion ratio calculated by standardization was 12%.

### Example 15: Preparation of Compound F-13a

Piperidine (8.0 equiv.) and Compound Ga (2.5 equiv.) were added to a mixture of Compound D (1.0 equiv.) and THF, and the mixture was stirred at 60 °C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a (yield: 31%) was obtained as a red solid by filtration under reduced pressure condition.

### Example 16: Preparation of Compound F-13a

Piperidine (8.0 equiv.) and Compound Ga (2.5 equiv.) were added to a mixture of Compound D (1.0 equiv.) and methanol, and stirred at 100 °C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a (yield: 5%) was obtained as a red solid by filtration under reduced pressure condition.

### Example 17: Preparation of Compound F-13a

To a mixture of Compound D (1.0 equiv.) and 1,4-dioxane, piperidine (8.0 equiv.) and Compound Ga (2.5 equiv.) were added and stirred at 100° C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a (yield: 12%) was obtained as a red solid by filtration under reduced pressure condition.

### Example 18: Preparation of Compound F-13a

Piperazine (8.0 equiv.) and Compound Ga (2.5 equiv.) were added to a mixture of Compound D (1.0 equiv.) and 1,4-dioxane, and stirred at 100 °C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a (yield: 6%) was obtained as a red solid by filtration under reduced pressure condition.

### Example 19: Preparation of Compound F-13a

Azocane (8.0 equiv.) and Compound Ga (2.5 equiv.) were added to a mixture of Compound D (1.0 equiv.) and 1,4-dioxane, and stirred at 25 °C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. Compound F-13a (yield: 22%) was obtained as a red solid by filtration under reduced pressure condition.

### Example 20: Preparation of Compound F-13a

Azocane (8.0 equiv.) and Compound Ga (2.5 equiv.) were added to a mixture of Compound D (1.0 equiv.) and 1,4-dioxane, and the mixture was stirred at 100 °C for 16 hours under nitrogen condition. After the mixture was concentrated under reduced pressure condition, CHCl₃ (400 mL) was added to the residue and washed with 0.1M aqueous hydrochloric acid solution (80 mL x 2). The organic layer was separated, dried over anhydrous Na₂SO₄, filtered and concentrated under reduced pressure condition. The residue was triturated with methanol (30 mL)/MTBE (30 mL) at 20 °C. A red solid Compound F-13a (yield: 20%) was obtained by filtration under reduced pressure condition.

### 3.2. Preparation of Compound F-13a by coupling of Compound D and Compound Gb

D-Gb corresponding to the compound represented by Formula 3 was prepared by coupling the Compound D of Example 2 with the Compound Gb of Example 6, and Compound F-13a was prepared therefrom.

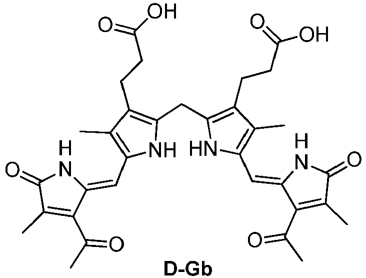

### Example 21: Preparation of Compound D-Gb

To a mixture of Compound D (7.4 mg, 0.02 mmol, 1.0 equiv.) and Compound Gb (11.0 mg, 0.08 mmol, 2.0 equiv.) in 1,4-dioxane (1 mL), azepane (24 mg, 0.24 mmol, 6.0 equiv.) was added and stirred at 60 °C for 12 hours. After concentration under reduced pressure condition, CHCl₃ (50 mL) was added to the residue and washed with 0.2 N HCl aqueous solution (50 mL x 2). The organic layer was separated, dried over anhydrous Na₂SO₄, filtered and concentrated under reduced pressure condition. The residue was purified by silica gel chromatography to yield Compound D-Gb as a yellow solid (16 mg, 0.043 mmol, yield: 22%).
C₃₃H₃₆N₄O₈ m/z [M+H]⁺ = 618

### 3.3. Preparation of Compound F-13a by coupling of Compound D and Compound Gc

D-Gc corresponding to the compound represented by Formula 3 was prepared by coupling the Compound D of Example 2 with the Compound Gc of Example 7, and Compound F-13a was prepared therefrom.

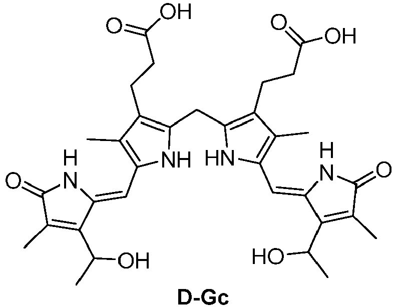

### Example 22: Preparation of Compound D-Gc

To a mixture of Compound D (3.66 mg, 0.0097 mmol, 1.0 equiv.) and 1,4-dioxane (0.5 mL), azepane (0.066 mL, 0.058 mmol, 6.0 equiv.) and Compound Gc (6.9 mg, 0.048 mmol, 5.0 equiv.) were added and stirred at 45 °C for 18 hours under nitrogen condition. It was confirmed by LCMS that Compound D-Gc was produced.
C₃₃H₄₀N₄O₈ m/z [M+H]⁺ = 621

### 3.4. Preparation of Compound F-13a by coupling of Compound D and Compound Gd

D-Gd corresponding to the compound represented by Formula 3 was prepared by coupling the Compound D of Example 2 with the Compound Gd of Example 8, and Compound F-13a was prepared therefrom.

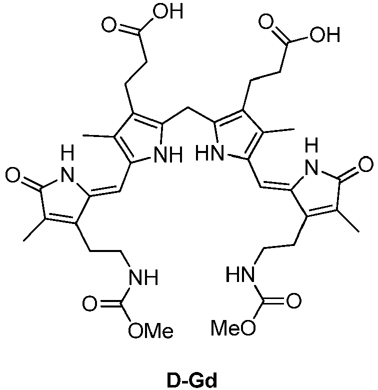

### Example 23: Preparation of Compound D-Gd

To a mixture of Compound D (37.0 mg, 0.099 mmol, 1.0 equiv.) and Compound Gd (49.0 mg, 0.247 mmol, 2.5 equiv.) in 1,4-dioxane (3 mL), azepane (59.4 mg, 0.593 mmol, 6.0 equiv.) was added and stirred at 60 °C for 12 hours. After concentration under reduced pressure condition, CHCl₃ (50 mL) was added to the residue and washed with 0.2 N HCl aqueous solution (50 mL x 2). The organic layer was separated, dried over anhydrous Na₂SO₄, filtered and concentrated under reduced pressure condition. The residue was purified by silica gel chromatography to yield Compound D-Gd as a red solid.
C₃₇H₄₆N₆O₁₀ m/z [M+H]⁺ = 736

### Example 24: Preparation of Compound D-Gda from Compound D-Gd

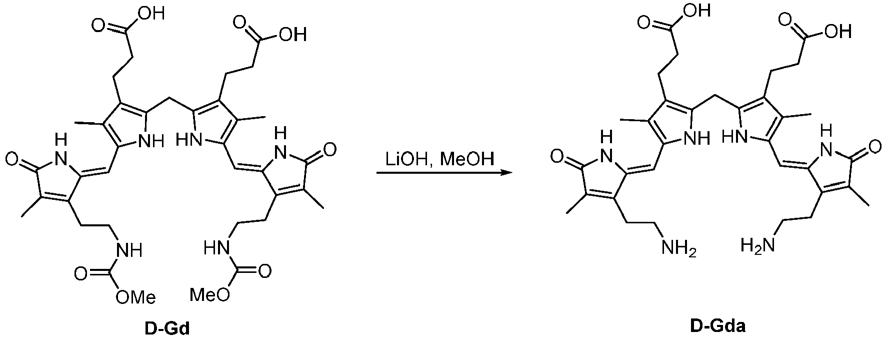

After dissolving the purified Compound D-Gd (6.6 mg, 0.022 mmol, 1.0 equiv.) in MeOH (3 mL), 1M LiOH aqueous solution (0.132 ml, 6.0 equiv.) was added thereto. After stirring the mixture at 60 °C for 4 hours, it was diluted in CHCl₃ (50 mL) and washed with 0.2 N HCl aqueous solution (50 mL x 2). The organic layer was separated, dried over anhydrous Na₂SO₄, filtered and concentrated under reduced pressure condition to yield an orange Compound D-Gda.
C₃₃H₄₂N₆O₆ m/z [M+H]⁺ = 620

### 3.5. Preparation of Compound F-13a by coupling of Compound D and Compound Ge

D-Ge corresponding to the compound represented by Formula 3 was prepared by coupling the Compound D of Example 2 with the Compound Ge of Example 10, and Compound F-13a was prepared therefrom.

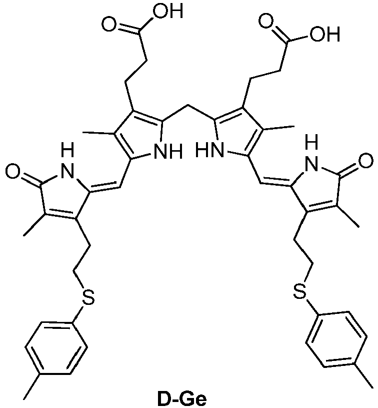

### Example 25: Preparation of Compound D-Ge

To a mixture of Compound D (36.0 mg, 0.096 mmol, 1.0 equiv.) in 1,4-dioxane (5 mL), azepane (65.6 µL, 0.58 mmol, 6.0 equiv.) was added and stirred at 25 °C for 10 minutes. Compound Ge (23.8 mg, 0.096 mmol, 1.0 equiv.) was added to the mixture and stirred at 80 °C for 12 hours. After concentration under reduced pressure condition, CHCl₃ (40 mL) was added to the residue and washed with 0.2M aqueous hydrochloric acid solution (20 mL x 2). The organic layer was separated, dried over anhydrous Na₂SO₄, filtered and concentrated under reduced pressure condition. The residue was triturated with DCM (5 mL)/Hexanes (20 mL) and filtered under reduced pressure condition to yield Compound D-Ge as a brown solid (30 mg, 0.036 mmol, yield: 37%).

C₄₇H₅₂N₄O₆S₂ m/z [M+H]⁺ = 834

### Example 26: Preparation of Compound F-13a from Compound D-Ge

A mixture of Compound D-Ge (26 mg, 0.031 mmol, 1.0 equiv.) in 1,4-dioxane (8 mL) was cooled to 0 °C, then m-CPBA (6.9 mg, 0.04 mmol, 1.3 equiv.) was added and stirred at 0 °C for 1 hour. To this mixture, NaHSO₃ aqueous solution (10 mL) was added, followed by extraction with CHCl₃ (20 mL x 2). The mixed organic layer was dried over anhydrous Na₂SO₄, filtered and concentrated under reduced pressure condition. After dissolving the residue in DMF (8 mL), pyridine (3 ml) was added, and the mixture was refluxed for 2 hours. It was confirmed through LCMS that Compound F-13a was produced.
C₃₃H₃₆N₄O₆ m/z [M+H]⁺ = 585

### 3.6. Preparation of Compound F-13a by coupling of Compound D and Compound Gf

D-Gf corresponding to the compound represented by Formula 3 was prepared by coupling the Compound D of Example 2 with the Compound Gf of Example 12, and Compound F-13a was prepared therefrom.

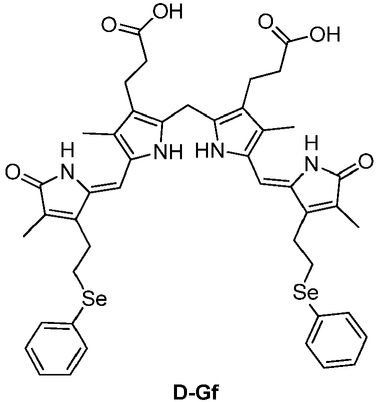

### Example 27: Preparation of Compound D-Gf

To a mixture of Compound D (37.3 mg, 0.099 mmol, 1.0 equiv.) in 1,4-dioxane (10 mL), azepane (68.1 µL, 0.58 mmol, 6.0 equiv.) was added and stirred at 25 °C for 10 minutes. Compound Gf (55.9 mg, 0.20 mmol, 2.0 equiv.) was added to the mixture and stirred at 80 °C for 14 hours. After concentration under reduced pressure condition, CHCl₃ (60 mL) was added to the residue and washed with 0.2M aqueous hydrochloric acid solution (40 mL x 2). The organic layer was separated, dried over anhydrous Na₂SO₄, filtered and concentrated under reduced pressure condition. The residue was triturated with EtOAc (5 mL)/Hexanes (50 mL) to yield Compound D-Gf as a red solid by filtration under reduced pressure condition (20 mg, 0.022 mmol, yield: 22%).
C₄₅H₄₈N₄O₆Se₂ m/z [M+H]⁺ = 900

### Example 28: Preparation of Compound F-13 from Compound D-Gf

To a mixture of D-Gf (15 mg, 0.017 mmol, 1.0 equiv.) in THF (2 mL), HOAc (3.0 mg, 0.050 mmol, 3.0 equiv.) and H₂O₂ (7.7 mg, 0.068 mmol, 4.0 equiv.) were added. Then, the mixture was stirred at 25 °C for 2 hours. It was confirmed by LCMS that Compound F-13a was produced.
C₃₃H₃₆N₄O₆ m/z [M+H]⁺ = 585

### 3.7. Preparation of Compound F-13a by coupling Compound C and Compound Ga

Compound C-Ga corresponding to the compound represented by Formula 3 was prepared by coupling the Compound C of Example 1 with the Compound Ga of Example 5, and Compound F-13a was prepared therefrom.

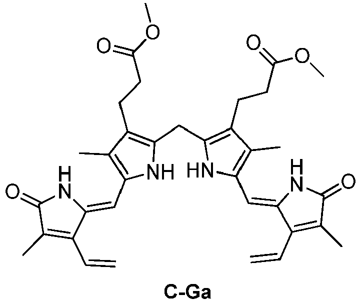

### Example 29: Preparation of Compound C-Ga

To a mixture of Compound C (392.14 mg, 0.97 mmol, 1.0 equiv.) and Compound Ga (300 mg, 2.44 mmol, 2.5 equiv.) in 1,4-dioxane (6 mL), piperidine (0.67 mL, 5.95 mmol, 6.1 equiv.) was added and stirred at 25 °C for 16 hours under nitrogen condition. CHCl₃ (60 mL) was added to the mixture and washed with 0.2 M HCl aqueous solution (20 mL x 2). The separated organic layer was dried over anhydrous Na₂SO₄, filtered and concentrated under reduced pressure condition. The residue was triturated with MeOH (5 mL) and filtered under reduced pressure to yield Compound C-Ga as a red solid (307 mg, 0.50 mmol, yield: 51%).

¹H NMR (400 MHz, DMSO-*d₆*) δ 10.50 (s, 2H), 10.00 (s, 2H), 6.85 (dd, *J* = 17.6, 12.0 Hz, 2H), 6.07 (s, 2H), 5.65 - 5.60 (m, 4H), 3.96 (s, 2H), 3.42 (s, 6H), 2.50 - 2.43 (m, overlap with DMSO-*d*₆'s signal, 4H), 1.98 (s, 6H), 1.92 - 1.88 (m, 10H).
C₃₅H₄₀N₄O₆ m/z [M+H]⁺ = 613

### Example 30: Preparation of Compound C-Ga

Pyrrolidine (8.0 equiv.) and Compound Ga (2.5 equiv.) were added to a mixture of Compound C (1.0 equiv.) and 1,4-dioxane, and the mixture was stirred at 20 °C for 16 hours under nitrogen condition. A reaction conversion ratio was measured by liquid chromatography mass spectrometry (LCMS), and the reaction conversion ratio calculated by standardization was 10%.

### Example 31: Preparation of Compound C-Ga

Pyrrolidine (8.0 equiv.) and Compound Ga (2.5 equiv.) were added to a mixture of Compound C (1.0 equiv.) and 1,4-dioxane, and stirred at 100 °C for 1 hour under nitrogen condition. A reaction conversion ratio was measured by liquid chromatography mass spectrometry (LCMS), and the reaction conversion ratio calculated by standardization was 23%.

### Example 32: Preparation of Compound C-Ga

Piperazine (8.0 equiv.) and Compound Ga (2.5 equiv.) were added to a mixture of Compound C (1.0 equiv.) and 1,4-dioxane, and stirred at 20 °C for 16 hours under nitrogen condition. A reaction conversion ratio was measured by liquid chromatography mass spectrometry (LCMS), and the reaction conversion ratio calculated by standardization was 14%.

### Example 33: Preparation of Compound C-Ga

Piperazine (8.0 equiv.) and Compound Ga (2.5 equiv.) were added to a mixture of Compound C (1.0 equiv.) and 1,4-dioxane, and stirred at 100 °C for 3 hours under nitrogen condition. A reaction conversion ratio was measured by liquid chromatography mass spectrometry (LCMS), and the reaction conversion ratio calculated by standardization was 31%.

### Example 34: Preparation of Compound C-Ga

Morpholine (8.0 equiv.) and Compound Ga (2.5 equiv.) were added to a mixture of Compound C (1.0 equiv.) and 1,4-dioxane, and stirred at 25 °C for 16 hours under nitrogen condition. A reaction conversion ratio was measured by liquid chromatography mass spectrometry (LCMS), and the reaction conversion ratio calculated by standardization was 4%.

### Example 35: Preparation of Compound C-Ga

Morpholine (8.0 equiv.) and Compound Ga (2.5 equiv.) were added to a mixture of Compound C (1.0 equiv.) and 1,4-dioxane, and stirred at 100 °C for 16 hours under nitrogen condition. A reaction conversion ratio was measured by liquid chromatography mass spectrometry (LCMS), and the reaction conversion ratio calculated by standardization was 18%.

### Example 36: Preparation of Compound C-Ga

Piperidine (8.0 equiv.) and Compound Ga (2.5 equiv.) were added to a mixture of Compound C (1.0 equiv.) and 1,4-dioxane, and stirred at 20 °C for 16 hours under nitrogen condition. A reaction conversion ratio was measured by liquid chromatography mass spectrometry (LCMS), and the reaction conversion ratio calculated by standardization was 27%.

### Example 37: Preparation of Compound C-Ga

Azepane (8.0 equiv.) and Compound Ga (2.5 equiv.) were added to a mixture of Compound C (1.0 equiv.) and 1,4-dioxane, and stirred at 100 °C for 16 hours under nitrogen condition. A reaction conversion ratio was measured by liquid chromatography mass spectrometry (LCMS), and the reaction conversion ratio calculated by standardization was 21%.

### Example 38: Preparation of Compound C-Ga

Azocane (8.0 equiv.) and Compound Ga (2.5 equiv.) were added to a mixture of Compound C (1.0 equiv.) and 1,4-dioxane, and stirred at 100 °C for 16 hours under nitrogen condition. A reaction conversion ratio was measured by liquid chromatography mass spectrometry (LCMS), and the reaction conversion ratio calculated by standardization was 22%.

### Example 39: Preparation of Compound C-Ga

Azepane (8.0 equiv.) and Compound Ga (2.5 equiv.) were added to a mixture of Compound C (1.0 equiv.) and 1,4-dioxane, and stirred at 100 °C for 16 hours under nitrogen condition. A reaction conversion ratio was measured by liquid chromatography mass spectrometry (LCMS), and the reaction conversion ratio calculated by standardization was 13%.

### Example 40: Preparation of Compound F-13a from Compound C-Ga

To a mixture of Compound C-Ga (100 mg, 0.16 mmol, 1.0 equiv.) in methanol (2.5 mL) and water (0.5 mL), LiOH·H₂O (41.09 mg, 0.98 mmol, 6.0 equiv.) was added and stirred at 60 °C for 2 hours. 1 M HCl aqueous solution was added dropwise to the mixture to adjust the pH to 2-3. At this time, the produced solid was filtered under reduced pressure to obtain a brown solid. The solid was triturated with MeOH (5 mL) to yield Compound F-13a as a red solid (67 mg, 0.11 mmol, yield: 70%).

¹H NMR (400 MHz, DMSO-*d₆*) δ 10.58 (brs, 2H), 6.79 (dd, *J* = 17.2, 11.6 Hz, 2H), 6.05 (s, 2H), 5.63 - 5.58 (m, 4H), 3.93 (s, 2H), 2.50 (m, overlap with DMSO-*d*₆'s signal, 4H), 2.05 (m, 4H), 1.98 (s, 6H), 1.90 (s, 4H).

### 3.8. Preparation of Compound F-13a by coupling of Compound C1 and Compound Ga

Compound C1 of Example 3 and Compound Ga of Example 5 were coupled to prepare Compound C1-Ga corresponding to the compound represented by Formula 3, and Compound F-13a was prepared therefrom.

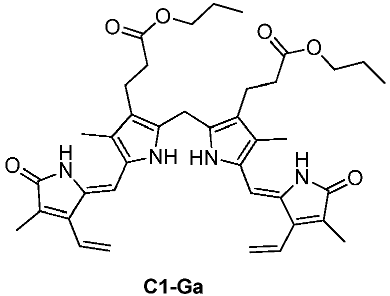

### Example 41: Preparation of Compound C1-Ga

To a mixture of Compound C1 (100 mg, 0.21 mmol, 1.0 equiv.) in 1,4-dioxane (1.5 mL), piperidine (0.18 g, 2.1 mmol, 10.0 equiv.) and Compound Ga (78.5 mg, 0.64 mmol, 3.0 equiv.) were added and stirred at 101 °C for 16 hours under nitrogen condition. CHCl₃ (40 mL) was added to the mixture and washed with 0.1 M HCl aqueous solution (25 mL x 2). The mixed organic layer was washed with brine (25 mL), dried over anhydrous Na₂SO₄, filtered and concentrated under reduced pressure condition. The residue was purified by silica gel chromatography to yield Compound C1-Ga as a red solid (32.3 mg, 0.048 mmol, yield: 23%).

¹H NMR (400 MHz, DMSO-*d*₆) δ 10.53 (brs, 2H), 10.01 (brs, 2H), 6.81 (dd, *J* = 17.6, 11.6 Hz, 2H), 6.06 (s, 2H), 5.65 - 5.60 (m, 4H), 3.96 (s, 2H), 3.76 (t, *J* = 6.8 Hz, 4H), 2.50 - 2.43 (m, overlap with DMSO-*d*₆'s signal, 4H), 1.97 (s, 6H), 1.92 (s, 6H), 1.82 (t, *J* = 8.0 Hz, 4H), 1.42 (q, *J* = 7.2 Hz, 4H), 0.75 (t, *J* = 7.2 Hz, 6H).
C₃₉H₄₈N₄O₆ m/z [M+H]⁺ = 669.5

### Example 42: Preparation of Compound F-13a from Compound C1-Ga

To a mixture of Compound C1-Ga (60 mg, 0.089 mmol, 1.0 equiv.) in methanol (1 mL), a mixture of LiOH·H₂O (22.14 mg, 0.53 mmol, 6.0 equiv.) in water (0.5 mL) was added and stirred at 60 °C for 2 hours under nitrogen condition. CHCl₃ (25 mL) was added to the mixture followed by addition of 0.1 M HCl aqueous solution (15 mL) to adjust the pH with acid. The mixed organic layer was dried over anhydrous Na₂SO₄, filtered and concentrated under reduced pressure condition. DCM/Hexanes was added to the residue, and the precipitated solid was filtered under reduced pressure to yield Compound F-13a as an orange solid (10.4 mg, 0.025 mmol, yield: 20%).
C₃₃H₃₆N₄O₆ m/z [M+H]⁺ = 585

### 3.9. Preparation of Compound F-13a by coupling of Compound C2 and Compound Ga

Compound C2 of Example 4 and Compound Ga of Example 5 were coupled to prepare Compound C2-Ga corresponding to the compound represented by Formula 3, and Compound F-13a was prepared therefrom.

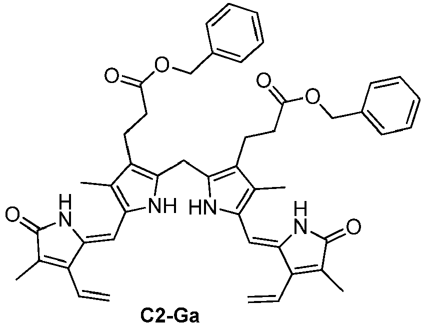

### Example 43: Preparation of Compound C2-Ga

To a mixture of Compound C2 (100 mg, 0.17 mmol, 1.0 equiv.) in 1,4-dioxane (2 mL), piperidine (0.14 g, 1.7 mmol, 10.0 equiv.) and Compound Ga (59.1 mg, 0.48 mmol, 3.0 equiv.) were added and stirred at 101 °C for 12 hours under nitrogen condition. CHCl₃ (40 mL) was added to the mixture and washed with 0.1 M HCl aqueous solution (30 mL x 2). The mixed organic layer was washed with brine (40 mL), dried over anhydrous Na₂SO₄, filtered and concentrated under reduced pressure condition. EtOAc/Hexanes was added to the residue and the precipitated dark orange solid was filtered to yield Compound C2-Ga (65 mg, 0.085 mmol, yield: 50%).

¹H NMR (400 MHz, DMSO-*d*₆) δ 10.52 (s, 2H), 10.03 (s, 2H), 7.30 - 7.19 (m, 10H), 6.75 (dd, *J* = 17.6, 11.6 Hz, 2H), 6.06 (s, 2H), 5.65 - 5.57 (m, 4H), 4.91 (s, 4H), 3.97 (s, 2H), 2.50 - 2.45 (m, overlap with DMSO-*d*₆'s signal, 4H), 2.02 - 1.97 (m, 4H), 1.95 (s, 6H), 1.91 (s, 6H).
C₄₇H₄₈N₄O₆ m/z [M+H]⁺ = 765.6

### Example 44: Preparation of Compound F-13a from Compound C2-Ga

To a mixture of Compound C2-Ga (30 mg, 0.039 mmol, 1.0 equiv.) in THF (1 mL), Pd/C (2.5 mg, 10 mol%) was added under nitrogen condition. The mixture was degassed under vacuum condition and filled with H₂ several times. The mixture was stirred at 25 °C for 4 hours under H₂ condition. It was confirmed by LCMS that Compound F-13a was produced.
C₃₃H₃₆N₄O₆ m/z [M+H]⁺ = 585

### 4. PEGylation of compound represented by Formula 3

Compound FP-13a was prepared by PEGylating F-13a corresponding to the compound represented by Formula 3 (Examples 45 to 50).

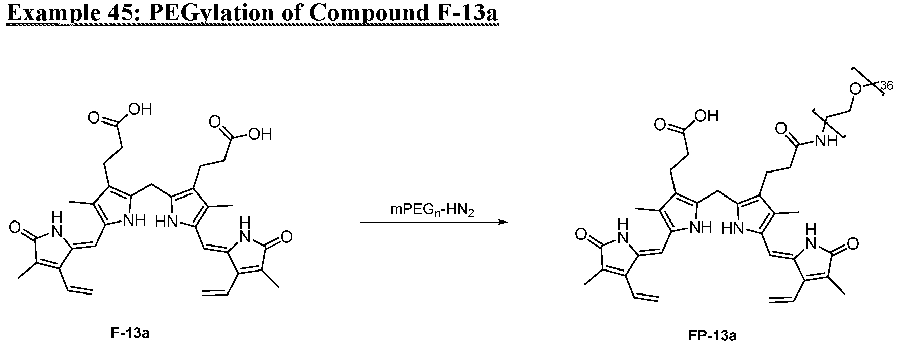

Compound F-13a (90 mg, 0.15 mmol, 1.0 equiv.) was dissolved in DMSO (5 mL) and then stirred at 25 °C for 15 minutes. A mixture of CDI (37.44 mg, 0.23 mmol, 1.5 equiv.) in DMSO (2 mL) was added dropwise to the above mixture, and stirred at 25 °C for 2 hours. To this mixture, a mixture of mPEG₃₆-NH₂ (99.56 mg, 0.061 mmol, 0.4 equiv.) in DMSO (1 mL) was added and stirred at 25 °C for 4 hours. Na₂CO₃ aqueous solution (100 mL) was added to the mixture and extracted with CHCl₃ (50 mL x 2). The separated organic layer was dried over anhydrous Na₂SO₄, filtered and concentrated under reduced pressure condition. The residue was triturated with MTBE (50 mL), and the resulting solid was dissolved in CHCl₃ (50 mL) and then concentrated under reduced pressure condition. H₂O (10 mL) was added to the resulting oily residue, followed by freeze-drying and purification with sephadex LH-20 to yield Compound FP-13a in the form of red solid (35 mg, 0.016 mmol, yield: 26%).
C₁₀₆H₁₈₃N₅O₄₁ m/z [M]⁺ = 2182

### Example 46: PEGylation of F-13a

A mixture of Compound F-13a (1.0 equiv.) in DMA was stirred at 25 °C for 15 minutes. A mixture of CDI (1.2 equiv.) in DMA was added dropwise to the above mixture. After stirring at 25°C for 2 hours, a mixture of mPEG₃₆-NH₂ (0.4 equiv.) in DMA was added thereto, and stirred at 25 °C for 16 hours. A reaction conversion ratio was measured by liquid chromatography mass spectrometry (LCMS), and the reaction conversion ratio calculated by standardization was 34%.

### Example 47: PEGylation of F-13a

A mixture of Compound F-13a (1.0 equiv.) in DMSO was stirred at 25 °C for 15 minutes. A mixture of CMPI (1.2 equiv.) in DMSO was added dropwise to the above mixture. After stirring at 25 °C for 1 hour, a mixture of mPEG₃₆-NH₂ (0.4 equiv.) in DMSO was added thereto, and stirred at 0 °C for 4 hours. A reaction conversion ratio was measured by liquid chromatography mass spectrometry (LC-MS), and the reaction conversion ratio calculated by standardization was 26%.

### Example 48: PEGylation of F-13a

A mixture of Compound F-13a (1.0 equiv.) in DMF was stirred at 25 °C for 15 minutes. A mixture of EDCI (1.1 equiv.), pentafluorophenol (1.1 equiv.) and DIPEA (1.1 equiv.) in DMF was added dropwise to the above mixture. After stirring at 25 °C for 2 hours, a mixture of mPEG₃₆-NH₂ (0.4 equiv.) in DMF was added thereto, and stirred at 25 °C for 3 hours. A reaction conversion ratio was measured by liquid chromatography mass spectrometry (LC-MS), and the reaction conversion ratio calculated by standardization was 10%.

### Example 49: PEGylation of F-13a

A mixture of Compound F-13a (1.0 equiv.) in DCM/DMA was stirred at 25 °C for 15 minutes. A mixture of DCC (1.0 equiv.) and HOAt (0.2 equiv.) in DCM/DMA was added to the above mixture. After stirring at 25 °C for 2 hours, a mixture of mPEG₃₆-NH₂ (0.4 equiv.) in DCM/DMA was added thereto, and stirred at 25°C for 16 hours. A reaction conversion ratio was measured by liquid chromatography mass spectrometry (LC-MS), and the reaction conversion ratio calculated by standardization was 15%.

### Example 50: PEGylation of F-13a

A mixture of Compound F-13a (1.0 equiv.) in DMA was stirred at 25°C for 15 minutes. A mixture of DCC (1.5 equiv.) in DMA was added dropwise to the above mixture. After stirring at 25 °C for 2 hours, a mixture of mPEG₃₆-NH₂ (0.4 equiv.) in DMA was added thereto, and stirred at 25°C for 16 hours. A reaction conversion ratio was measured by liquid chromatography mass spectrometry (LC-MS), and the reaction conversion ratio calculated by standardization was 18%.

## Claims

1. A method for synthesizing bilirubin, comprising preparing a compound represented by Formula 3 below by coupling a compound represented by Formula 1 below with a compound represented by Formula 2 below: 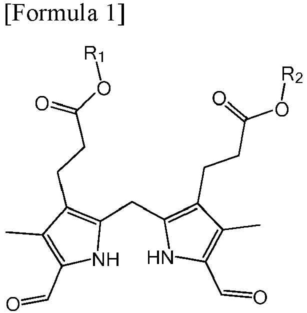 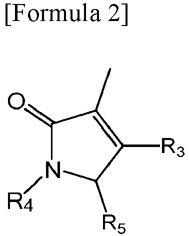 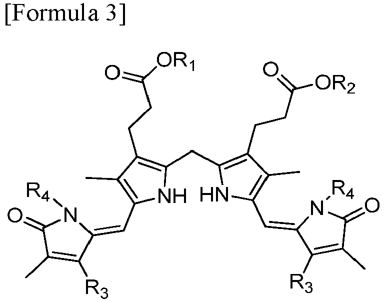 (wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₃ is a vinyl or acetyl group, or an ethyl group substituted with a hydroxyl group, carbamate, selenide or sulfide; R₄ is hydrogen or a nitrogen protecting group; and R₅ is hydrogen, a tosyl or mesyl group).

2. The method according to claim 1, further comprising reacting the compound represented by Formula 3 with polyethylene glycol (PEG).

3. The method according to claim 1, wherein the compound represented by Formula 1 is reacted with polyethylene glycol (PEG) and then coupled with the compound represented by Formula 2.

4. The method according to claim 1, further comprising dimerizing a compound represented by Formula 7 below to prepare the compound represented by Formula 1: 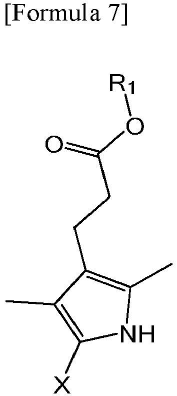 (wherein, R₁ is the same as R₁ in Formula 1, X is an arylalkyl ester group having 8 to 20 carbon atoms, -CH₂OH, -COOH, a halogen atom or hydrogen).

5. The method according to claim 1, further comprising oxidizing a compound represented by Formula 9 below to prepare the compound represented by Formula 2: 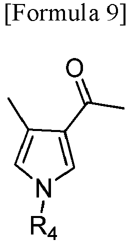 (wherein, R₄ is the same as R₄ in Formula 2).

6. The method according to claim 1, further comprising reducing an acetyl group of a compound represented by Formula 10 below to prepare the compound represented by Formula 2: 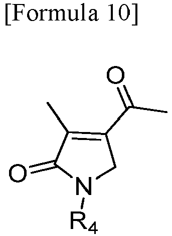 (wherein, R₄ is the same as R₄ in Formula 2).

7. The method according to claim 1, further comprising dehydrating a hydroxyl group of a compound represented by Formula 11 below to prepare the compound represented by Formula 2: 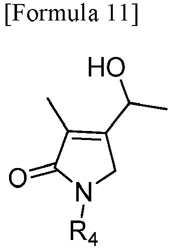 (wherein, R₄ is the same as R₄ in Formula 2).

8. The method according to claim 1, further comprising processing a compound represented by Formula 12 through cyclization and halogen removal to prepare the compound represented by Formula 2: 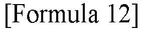 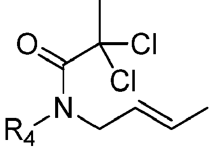 (wherein, R₄ is the same as R₄ in Formula 2).

9. The method according to claim 1, further comprising oxidizing and carbamating a compound represented by Formula 13 below to prepare the compound represented by Formula 2: 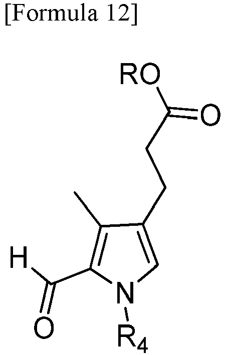 (wherein, R is an alkyl group having 1 to 12 carbon atoms, and R₄ is the same as R₄ in Formula 2).

10. The method according to claim 1, further comprising cyclizing a compound represented by Formula 14 below to prepare the compound represented by Formula 2: 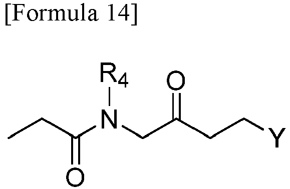 (wherein, Y is selenide, and R₄ is the same as R₄ in Formula 2).

11. The method according to claim 1, further comprising oxidizing a compound represented by Formula 15 below to prepare the compound represented by Formula 2: 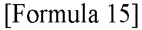 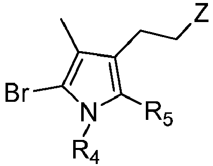 (wherein, Z is sulfide, R₄ and R₅ are the same as R₄ and R₅ in Formula 2).

12. The method according to claim 1, further comprising preparing bilirubin from the compound represented by Formula 3.

13. The method according to claim 1, wherein the step of preparing a compound is carried out in the presence of a base selected from the group consisting of piperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidine-3-ol, piperazine, 2,6-dimethylpiperazine, 1-benzyl piperazine, 1-isopropyl piperazine, 2-ethyl piperazine, morpholine, 4-methyl morpholine, 2,6-dimethyl morpholine, ethyl morpholine, azepane, 2-methyl azepane, 4-methyl azepane, 2,2,7,7-tetramethyl azepane, 1,2,2-trimethyl azepane, 1,2-dimethylazepane, 2,7-dimethyl azepane, methyl azepane-4-carboxylate, azocane, 2-methyl azocane, 1,2-dimethyl azocane, 1,2,2-trimethyl azocane, methyl azocane-2-carboxylate, 1-methyl azocane and 2(2-methylphenyl)azocane.

14. The method according to claim 1, wherein the step of preparing a compound is carried out in the presence of a solvent selected from the group consisting of water, alcohols, ethers, ketones, aliphatic hydrocarbons, aromatic hydrocarbons, halogenated hydrocarbons, alkoxies, nitriles and amides.

15. The method according to claim 1, wherein the step of preparing a compound is carried out at -20 to 200 °C.

16. The method according to claim 1, wherein the step of preparing a compound is carried out for 0.5 to 120 hours.

17. The method according to claim 13, wherein the base is added in an amount of 2 to 20 moles based on 1 mole of the compound represented by Formula 1.
